(19) Europäisches Patentamt
European Patent Office
Office européen des brevets

(11) **EP 3 822 369 A1**

(12) **EUROPEAN PATENT APPLICATION**
published in accordance with Art. 153(4) EPC

(43) Date of publication:
**19.05.2021 Bulletin 2021/20**

(21) Application number: **19833075.5**

(22) Date of filing: **10.07.2019**

(51) Int Cl.:
***C12Q 1/6886*** (2018.01)

(86) International application number:
**PCT/CN2019/095415**

(87) International publication number:
**WO 2020/011195 (16.01.2020 Gazette 2020/03)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(30) Priority: **13.07.2018 CN 201810771196**

(71) Applicant: **Lisen Imprinting Diagnostics, Inc.
Dover, Delaware 19901 (US)**

(72) Inventors:
• **CHENG, Tong**
 **Wuxi, Jiangsu 214000 (CN)**
• **ZHOU, Ning**
 **Wuxi, Jiangsu 214000 (CN)**

(74) Representative: **Grund, Martin et al
Grund Intellectual Property Group
Patentanwalt und Solicitor PartG mbB
Postfach 44 05 16
80754 München (DE)**

(54) **GRADING MODEL USED FOR DETECTING DEGREE TO WHICH LUNG TUMORS ARE BENIGN AND MALIGNANT, AND APPLICATION THEREOF**

(57) A grading model for detecting the degree of benignity/malignancy of a lung tumor and its applications are disclosed. The model grades the changes in an imprinted gene in a lung tumor by calculating an expressed quantity of the imprinted gene with a loss of imprinting, an expressed quantity of the imprinted gene with a copy number variation, and a total expressed quantity of the imprinted gene. The detection model and device disclosed herein enable intuitive observation of the expression of an imprinted gene with a loss of imprinting in a tissue or cell sample taken from a patient with a lung tumor. By labeling the imprinted gene in situ, changes in the imprinted gene can be objectively, intuitively, and precisely detected at an early stage. Moreover, a quantitative model is provided. Thus, the disclosure contributes greatly to the diagnosis of lung tumors.

FIG. 1

**Description**

**Technical Field**

The present disclosure pertains to the field of biotechnology, such as the field of genetic diagnosis, such as a grading model and its applications, such as a grading model for detecting the degree of benignity/malignancy of a lung tumor and its applications, such as a grading model for detecting the degree of benignity/malignancy of a lung tumor through a combination of imprinted genes and a device using the model.

**Description of Related Art**

Lung cancer, or pulmonary carcinoma, is the malignant tumor with the highest morbidity and mortality in the world. According to statistics of the World Health Organization (WHO), or more specifically World Cancer Report 2014, the year 2012 saw 1.82 million new cases of lung cancer worldwide, including 1.59 million deaths. In China, the same year saw 733 thousand newly diagnosed cases of lung cancer, including 610 thousand deaths, with lung cancer having the highest male morbidity and male mortality, the second highest female morbidity, and the highest female mortality of all cancers, according to the same report. The survival rate of a patient with lung cancer is closely related to the progression of the cancer. Patients with stage I lung cancer have a five-year survival rate as high as 70%-90%, whereas patients with stage IV lung cancer have a five-year survival rate of 10% at most. Early diagnosis and early treatment, therefore, are critical to saving the lives of patients with lung cancer. Recently, the early diagnosis rate of lung cancer has been raised from lower than 30% to 55%, thanks to the application of various new technologies such as low-dose spiral computed tomography (CT), positron emission tomography/CT (PET/CT), fluorescence bronchoscopy, and endobronchial ultrasound (EBUS). A large number of patients, however, are still diagnosed with lung cancer that has already developed to an advanced stage. A more sensitive and more accurate method for the early detection of lung cancer is hence needed urgently.

Cancer takes place when cells grow and/or divide in an uncontrolled manner resulting from an accumulation of epigenetic changes and genetic variations over time. In a conventional pathological diagnosis, a lung tumor is determined to be benign or malignant based on the variations in size, morphology, and structure of cells and tissue. With the development and advancement of molecular biology, more and more molecular detection techniques have been used to detect lung cancer. Analyses of the process of cancer development have shown that molecular changes (in epigenetics and genetics) occur far earlier than variations in cell morphology and tissue structure, and this is why molecular biology-based detection is more sensitive than other detection methods in detecting cancer at an early stage.

According to the above, the existing lung cancer diagnosis methods are in need of a new detection system and detection model that can be applied to a patient's biopsy samples to analyze lung cancer-related changes in a molecular marker on a cellular level, in order to provide more accurate prognosis and diagnosis information.

**Brief Summary of the Invention**

The present disclosure provides an imprinted gene grading model, a diagnostic method using the model, and other applications of the model.

To achieve the foregoing objective, the following technical solution is used:

The present disclosure provides an imprinted gene grading model that is applicable to a lung tumor. The model calculates changes in the following expressed quantities of an imprinted gene in a lung tumor and grades the expression state of the imprinted gene accordingly: an expressed quantity of the imprinted gene with a loss of imprinting, an expressed quantity of the imprinted gene with a copy number variation, and a total expressed quantity of the imprinted gene.

The imprinted gene may be any one, or a combination of at least two, of Z1, Z11, and Z16, wherein the imprinted gene Z1 is Gnas, the imprinted gene Z11 is Grb10, and the imprinted gene Z16 is Snrpn/Snurf.

Loss of imprinting refers to the activation (demethylation) of a previously silenced allele of an imprinted gene and is the most common and earliest epigenetic change in cancer and therefore a characteristic that can be used as a pathological marker. By contrast, loss of imprinting seldom occurs in a healthy cell.

The inventor of the present disclosure has found that a diagnostic sensitivity of at least 73.7% can be achieved for the diagnosis of lung tumors by calculating the aforesaid expressed quantities of any one of the imprinted genes Z1, Z11, and Z16 in a lung tumor, i.e., the expressed quantity of the imprinted gene with a loss of imprinting, the expressed quantity of the imprinted gene with a copy number variation, and the total expressed quantity of the imprinted gene.

In one embodiment of the present disclosure, any one of the imprinted genes Z1, Z11, and Z16 can be detected if only one imprinted gene is to be detected in preliminary detection.

In one embodiment of the present disclosure, either one of the imprinted genes Z1 and Z16 can be detected if only one imprinted gene is to be detected in preliminary detection.

In one embodiment of the present disclosure, the imprinted gene Z1 can be detected if only one imprinted gene is to be detected in preliminary detection.

The inventor has found in one embodiment of the present disclosure that a diagnostic sensitivity of 86.0% can be achieved for the diagnosis of lung tumors by detecting only the imprinted gene Z1, 73.7% by detecting only the imprinted gene Z11, and 78.9% by detecting only the imprinted gene Z16.

In one embodiment of the present disclosure, the model calculates the aforesaid expressed quantities of the to-be-detected imprinted gene in such a way that if a combination of two imprinted genes are to be detected, the combination may include any two of Z1, Z11, and Z16, preferably a combination of Z1 and Z16 or a combination of Z1 and Z11.

The inventor has found that diagnostic sensitivity can be increased by calculating the expressed quantities of two or more imprinted genes with a loss of imprinting, the expressed quantities of the two or more imprinted genes with a copy number variation, and the respective total expressed quantities of the two or more imprinted genes. A diagnostic sensitivity of at least 93.0% can be achieved for the diagnosis of lung cancer by detecting a combination of Z1 and Z16 or a combination of Z1 and Z11, and at least 91.2% by detecting a combination of Z11 and Z16.

In one embodiment of the present disclosure, the imprinted gene to be detected may further include any one, or a combination of at least two, of Z3, Z4, Z5, Z6, Z8, Z10, and Z13, wherein the imprinted gene Z3 is Peg10, the imprinted gene Z4 is Igf2r, the imprinted gene Z5 is Mest, the imprinted gene Z6 is Plagl1, the imprinted gene Z8 is Dcn, the imprinted gene Z10 is Gatm, and the imprinted gene Z13 is Sgce.

The inventor has found that detecting one, or a combination of at least two, of Z3, Z4, Z5, Z6, Z8, Z10, and Z13 in addition to one, or a combination of at least two, of Z1, Z11, and Z16 for a joint diagnosis not only helps increase the accuracy of detection, but also prevents false positive results thanks to the assistance of additional probes in making the diagnosis, thereby further enhancing the accuracy of detection and allowing all the lung tumor samples under examination to be accurately graded and judged.

In one embodiment of the present disclosure, the model calculates the aforesaid expressed quantities of the to-be-detected imprinted gene by calculating the aforesaid expressed quantities of a combination of imprinted genes, namely a combination of the imprinted genes Z1, Z3, Z4, Z5, Z6, Z8, Z10, Z11, Z13, and Z16.

An imprinted gene is determined as having a loss of imprinting when two red/brown marks are present in the nucleus of the cell after the cell is stained with hematoxylin. An imprinted gene is determined as having a copy number variation when more than two red/brown marks are present in the nucleus of the cell after the cell is stained with hematoxylin. A copy number variation occurs when abnormal gene duplication takes place in a cancer cell such that the duplicated gene is expressed as having three or more copies of the original gene.

In one embodiment of the present disclosure, the following formulas are used to calculate the total expressed quantity of an imprinted gene, the expressed quantity of the imprinted gene with a loss of imprinting, and the expressed quantity of the imprinted gene with a copy number variation:

$$\text{total expressed quantity of the imprinted gene} = (b+c+d)/(a+b+c+d) \times 100\%;$$

$$\text{expressed quantity of the imprinted gene being normal} = b/(b+c+d) \times 100\%;$$

$$\text{expressed quantity of the imprinted gene with a loss of imprinting (LOI)} = c/(b+c+d) \times 100\%;$$

and

$$\text{expressed quantity of the imprinted gene with a copy number variation (CNV)} = d/(b+c+d) \times 100\%;$$

where a is the number of cells that, after being stained with hematoxylin, show no mark in the nucleus, meaning the imprinted gene is not expressed in the nucleus; b is the number of cells that, after being stained with hematoxylin, show one red/brown mark in the nucleus, meaning the imprinted gene is present in the nucleus; c is the number of cells that, after being stained with hematoxylin, show two red/brown marks in the nucleus, meaning the imprinted gene in the nucleus is affected by a loss of imprinting; and d is the number of cells that, after being stained with hematoxylin, show more than two red/brown marks in the nucleus, meaning the imprinted gene in the nucleus has a copy number variation.

In one embodiment of the present disclosure, the hematoxylin-stained marks are selected from but not limited to red marks and brown marks. Staining/marking in other colors is also feasible when calculating the expressed quantity of an imprinted gene with a loss of imprinting, the expressed quantity of the imprinted gene with a copy number variation, and the total expressed quantity of the imprinted gene.

In one embodiment of the present disclosure, in-situ hybridization is carried out by way of a probe, and the nuclei of cells are stained with hematoxylin for signal amplification. Each nucleus is then detected under a 40× or 60× microscope for the presence of the to-be-detected imprinted gene, for any loss of imprinting in the imprinted gene, and for any copy number variation in the imprinted gene. After that, the degree of benignity/malignancy of the tumor sample in question is determined by calculating the expressed quantity of the imprinted gene with a loss of imprinting, the expressed quantity of the imprinted gene with a copy number variation, and the total expressed quantity of the imprinted gene. As the section under detection is merely 10 μm thick, about 20% of the nuclei seen under the microscope are incomplete; in other words, the detection results may be partly false negative.

In one embodiment of the present disclosure, the expressed quantity of an imprinted gene with a loss of imprinting, the expressed quantity of the imprinted gene with a copy number variation, and the total expressed quantity of the imprinted gene are graded into five different grades. The foregoing expressed quantities of each of ten imprinted genes, namely Z1, Z3, Z4, Z5, Z6, Z8, Z10, Z11, Z13, and Z16, are obtained by counting at least 1200 cells in an area of a sample where the corresponding probe is the most clearly expressed, and the expressed quantities obtained are classified according to the five-grade scale.

In one embodiment of the present disclosure, the expressed quantities of the imprinted genes Z1, Z3, Z11, and Z16 with a loss of imprinting, the expressed quantities of the imprinted genes Z1, Z3, Z11, and Z16 with a copy number variation, and the respective total expressed quantities of the imprinted genes Z1, Z3, Z11, and Z16 are classified into the following five grades:

Grade 0: applicable when any one or at least two of the following conditions are met: the expressed quantity of the imprinted gene Z1, Z3, Z11, or Z16 with a loss of imprinting is less than 10%, the expressed quantity of the imprinted gene Z1, Z3, Z11, or Z16 with a copy number variation is less than 1%, and the total expressed quantity of the imprinted gene Z1, Z3, Z11, or Z16 is less than 20%;

Grade I: applicable when any one or at least two of the following conditions are met: the expressed quantity of the imprinted gene Z1, Z3, Z11, or Z16 with a loss of imprinting is 10%-20%, the expressed quantity of the imprinted gene Z1, Z3, Z11, or Z16 with a copy number variation is 1%-3%, and the total expressed quantity of the imprinted gene Z1, Z3, Z11, or Z16 is 20%-30%;

Grade II: applicable when any one or at least two of the following conditions are met: the expressed quantity of the imprinted gene Z1, Z3, Z11, or Z16 with a loss of imprinting is 20%-25%, the expressed quantity of the imprinted gene Z1, Z3, Z11, or Z16 with a copy number variation is 3%-5%, and the total expressed quantity of the imprinted gene Z1, Z3, Z11, or Z16 is 30%-40%;

Grade III: applicable when any one or at least two of the following conditions are met: the expressed quantity of the imprinted gene Z1, Z3, Z11, or Z16 with a loss of imprinting is 25%-30%, the expressed quantity of the imprinted gene Z1, Z3, Z11, or Z16 with a copy number variation is 5%-7%, and the total expressed quantity of the imprinted gene Z1, Z3, Z11, or Z16 is 40%-50%; and

Grade IV: applicable when any one or at least two of the following conditions are met: the expressed quantity of the imprinted gene Z1, Z3, Z11, or Z16 with a loss of imprinting is greater than 30%, the expressed quantity of the imprinted gene Z1, Z3, Z11, or Z16 with a copy number variation is greater than 7%, and the total expressed quantity of the imprinted gene Z1, Z3, Z11, or Z16 is greater than 50%;

wherein the aforesaid expressed quantities of any one of the imprinted genes Z1, Z3, Z11, and Z16 are independent of those of another.

In one embodiment of the present disclosure, the expressed quantities of the imprinted genes Z4, Z5, Z6, Z10, and Z13 with a loss of imprinting, the expressed quantities of the imprinted genes Z4, Z5, Z6, Z10, and Z13 with a copy number variation, and the respective total expressed quantities of the imprinted genes Z4, Z5, Z6, Z10, and Z13 are classified into the following five grades:

Grade 0: applicable when any one or at least two of the following conditions are met: the expressed quantity of the imprinted gene Z4, Z5, Z6, Z10, or Z13 with a loss of imprinting is less than 10%, the expressed quantity of the imprinted gene Z4, Z5, Z6, Z10, or Z13 with a copy number variation is less than 1%, and the total expressed quantity of the imprinted gene Z4, Z5, Z6, Z10, or Z13 is less than 15%;

Grade I: applicable when any one or at least two of the following conditions are met: the expressed quantity of the imprinted gene Z4, Z5, Z6, Z10, or Z13 with a loss of imprinting is 10%-15%, the expressed quantity of the imprinted gene Z4, Z5, Z6, Z10, or Z13 with a copy number variation is 1%-2%, and the total expressed quantity of the imprinted gene Z4, Z5, Z6, Z10, or Z13 is 15%-20%;

Grade II: applicable when any one or at least two of the following conditions are met: the expressed quantity of the imprinted gene Z4, Z5, Z6, Z10, or Z13 with a loss of imprinting is 15%-20%, the expressed quantity of the imprinted gene Z4, Z5, Z6, Z10, or Z13 with a copy number variation is 2%-4%, and the total expressed quantity of the imprinted gene Z4, Z5, Z6, Z10, or Z13 is 20%-30%;

Grade III: applicable when any one or at least two of the following conditions are met: the expressed quantity of the imprinted gene Z4, Z5, Z6, Z10, or Z13 with a loss of imprinting is 20%-25%, the expressed quantity of the imprinted gene Z4, Z5, Z6, Z10, or Z13 with a copy number variation is 4%-6%, and the total expressed quantity of the imprinted gene Z4, Z5, Z6, Z10, or Z13 is 30%-40%; and

Grade IV: applicable when any one or at least two of the following conditions are met: the expressed quantity of the imprinted gene Z4, Z5, Z6, Z10, or Z13 with a loss of imprinting is greater than 25%, the expressed quantity of the imprinted gene Z4, Z5, Z6, Z10, or Z13 with a copy number variation is greater than 6%, and the total expressed quantity of the imprinted gene Z4, Z5, Z6, Z10, or Z13 is greater than 40%;

wherein the aforesaid expressed quantities of any one of the imprinted genes Z4, Z5, Z6, Z10, and Z13 are independent of those of another.

Preferably, the expressed quantity of the imprinted gene Z8 with a loss of imprinting, the expressed quantity of the imprinted gene Z8 with a copy number variation, and the total expressed quantity of the imprinted gene Z8 are classified into the following five grades:

Grade 0: applicable when any one or at least two of the following conditions are met: the expressed quantity of the imprinted gene Z8 with a loss of imprinting is less than 20%, the expressed quantity of the imprinted gene Z8 with a copy number variation is less than 2%, and the total expressed quantity of the imprinted gene Z8 is less than 15%;

Grade I: applicable when any one or at least two of the following conditions are met: the expressed quantity of the imprinted gene Z8 with a loss of imprinting is 20%-25%, the expressed quantity of the imprinted gene Z8 with a copy number variation is 2%-4%, and the total expressed quantity of the imprinted gene Z8 is 15%-20%;

Grade II: applicable when any one or at least two of the following conditions are met: the expressed quantity of the imprinted gene Z8 with a loss of imprinting is 25%-30%, the expressed quantity of the imprinted gene Z8 with a copy number variation is 4%-7%, and the total expressed quantity of the imprinted gene Z8 is 20%-30%;

Grade III: applicable when any one or at least two of the following conditions are met: the expressed quantity of the imprinted gene Z8 with a loss of imprinting is 30%-35%, the expressed quantity of the imprinted gene Z8 with a copy number variation is 7%-10%, and the total expressed quantity of the imprinted gene Z8 is 30%-40%; and

Grade IV: applicable when any one or at least two of the following conditions are met: the expressed quantity of the imprinted gene Z8 with a loss of imprinting is greater than 35%, the expressed quantity of the imprinted gene Z8 with a copy number variation is greater than 10%, and the total expressed quantity of the imprinted gene Z8 is greater than 40%.

In one embodiment of the present disclosure, a device for detecting the degree of benignity/malignancy of a lung tumor is provided. The device uses the model described above and includes the following units:

(1) a sampling unit for obtaining a test sample;
(2) a probe designing unit for designing a primer, or probe, specific to the sequence of the imprinted gene to be detected;
(3) a detection unit for performing in-situ hybridization between the probe designed by unit (2) and the test sample; and
(4) an analysis unit for analyzing the expression of the imprinted gene through microscopic imaging;

wherein the analysis unit calculates and grades, via the foregoing model, the expressed quantity of the imprinted gene with a loss of imprinting, the expressed quantity of the imprinted gene with a copy number variation, and the total expressed quantity of the imprinted gene, and determines the degree of benignity/malignancy of the lung tumor in question according to the grades of the aforesaid expressed quantities of the imprinted gene.

An imprinted gene is determined as having a loss of imprinting when two red/brown marks are present in the nucleus of the cell after the cell is stained with hematoxylin. An imprinted gene is determined as having a copy number variation when more than two red/brown marks are present in the nucleus of the cell after the cell is stained with hematoxylin. A copy number variation occurs when abnormal gene duplication takes place in a cancer cell such that the duplicated gene is expressed as having three or more copies of the original gene.

The hematoxylin-stained marks are selected from but not limited to red marks and brown marks. Staining/marking in other colors is also feasible when calculating the total expressed quantity of an imprinted gene, the expressed quantity of the imprinted gene with a loss of imprinting, and the expressed quantity of the imprinted gene with a copy number variation.

The detection device can be used to intuitively observe, on a cellular or tissue level and as early as possible, the changes of an imprinted gene in a lung tumor so as to determine the degree of benignity/malignancy of the tumor, thereby allowing a patient with an early-stage lung tumor to have the best chance of treatment.

In one embodiment of disclosure, a method for detecting the degree of benignity/malignancy of a lung tumor is provided. The method uses the foregoing model or device and includes the following steps:

(1) obtaining a test sample;
(2) designing a primer, or probe, specific to the sequence of the imprinted gene to be detected;
(3) performing in-situ hybridization between the probe in step (2) and the test sample; and
(4) analyzing the expression of the imprinted gene through microscopic imaging so as to diagnose the degree of benignity/malignancy of the lung tumor in question;

wherein step (4) includes calculating and grading, via the foregoing model, the expressed quantity of the imprinted gene with a loss of imprinting, the expressed quantity of the imprinted gene with a copy number variation, and the total expressed quantity of the imprinted gene, and diagnosing the degree of benignity/malignancy of the lung tumor according to the grades of the expressed quantities of the imprinted gene with, respectively, a loss of imprinting and a copy number variation.

In one embodiment of the present disclosure, the test sample in step (1) is tissue and/or cells obtained from a human.

The test sample can be chosen by a person skilled in the art according to practical needs and without limitation, provided that the RNA in the test sample is fixed in time. The test sample stated herein may include any one, or a combination of at least two, of a paraffin section of a tissue, a lung needle biopsy sample, a biopsy sample obtained through bronchoscopy, a cell smear prepared from a bronchoalveolar lavage fluid, and a cell smear prepared from sputum.

The steps of obtaining a paraffin section of a tissue include obtaining a human tumor tissue sample, fixing the tissue with 10% neutral buffered formalin in time, embedding the tissue in paraffin, cutting a 10 $\mu$m-thick section out of the paraffin-embedded tissue, and making a tissue slide with a positively charged slide. As the section is only 10 $\mu$m thick, some of the nuclei seen under a microscope are incomplete, which leads to partly false negative results regarding gene deletion.

The steps of obtaining a lung needle biopsy sample include obtaining human cells with a biopsy needle and fixing the cells with 10% neutral buffered formalin in time.

The steps of obtaining a biopsy sample through bronchoscopy include obtaining a suspicious tissue/suspicious cells through a bronchoscope and fixing the tissue/cells with 10% neutral buffered formalin in time.

A biopsy sample obtained through bronchoscopy has its special advantage not only because the obtainment of the biopsy sample causes little, if any, harm to the patient and can be done with ease, but also because the bronchoscope allows the exact location of the sample to be known, which is not the case with the drawing of blood, which circulates through the entire body.

A cell smear prepared from sputum has its special advantage because the obtainment of a sputum sample causes no harm to the patient and can be done with ease.

In one embodiment of the present disclosure, the test sample may be any one, or a combination of at least two, of a lung needle biopsy sample, a biopsy sample obtained through bronchoscopy, a cell smear prepared from a bronchoalveolar lavage fluid, and a cell smear prepared from sputum.

In one embodiment of the present disclosure, the imprinted genes to be detected are Z1, Z3, Z4, Z5, Z6, Z8, Z10, Z11, Z13, and Z16, wherein the imprinted gene Z1 is Gnas, the imprinted gene Z3 is Peg10, the imprinted gene Z4 is Igf2r, the imprinted gene Z5 is Mest, the imprinted gene Z6 is Plagl1, the imprinted gene Z8 is Dcn, the imprinted gene Z10 is Gatm, the imprinted gene Z11 is Grb10, the imprinted gene Z13 is Sgce, and the imprinted gene Z16 is Snrpn/Snurf.

The imprinted genes Zl(Gnas), Z3(Peg10), Z4(Igf2r), Z5(Mest), Z6(Plagl1), Z8(Dcn), Z10(Gatm), Z11(Grb10), Z13(Sgce), and Z16(Snrpn/Snurf) are expressed to different degrees in a normal tumor cell or tissue, and their expressed quantities and imprinted states change significantly when the tumor cell or tissue turns malignant.

The probe is designed according to the imprinted gene to be detected, namely Z1, Z3, Z4, Z5, Z6, Z8, Z10, Z11, Z13, or Z16, i.e., Gnas, Peg10, Igf2r, Mest, Plagl1, Dcn, Gatm, Grb10, Sgce, or Snrpn/Snurf. More specifically, a sequence is selected from the intron of each of the aforesaid genes as the corresponding probe. The probes used in the embodiments described further below were designed by Advanced Cell Diagnostics.

In one embodiment of the present disclosure, the in-situ hybridization uses an RNAscope in-situ hybridization method.

In one embodiment of the present disclosure, the RNAscope in-situ hybridization method uses a single- or multiple-channel chromogenic reagent kit or a single- or multiple-channel fluorescent reagent kit, preferably a single-channel red/brown chromogenic reagent kit or a multiple-channel fluorescent reagent kit.

The multiple-channel chromogenic reagent kit or multiple-channel fluorescent reagent kit includes a two (or more)-channel chromogenic or fluorescent reagent kit. The two-channel chromogenic reagent kit or multiple-channel fluorescent reagent kit can use two imprinted gene probes to detect the joint expression of each corresponding imprinted gene and another gene or use more than two imprinted gene probes to detect the joint expression of each corresponding imprinted gene and a non-imprinted gene.

In one embodiment of the present disclosure, the following formulas are used to calculate the total expressed quantity of an imprinted gene, the expressed quantity of the imprinted gene with a loss of imprinting, and the expressed quantity of the imprinted gene with a copy number variation:

$$\text{total expressed quantity of the imprinted gene} = (b+c+d)/(a+b+c+d)\times100\%;$$

$$\text{expressed quantity of the imprinted gene being normal} = b/(b+c+d)\times100\%;$$

$$\text{expressed quantity of the imprinted gene with a loss of imprinting (LOI)} = c/(b+c+d)\times100\%;$$

and

$$\text{expressed quantity of the imprinted gene with a copy number variation (CNV)} = d/(b+c+d)\times100\%;$$

where a is the number of cells that, after being stained with hematoxylin, show no mark in the nucleus, meaning the imprinted gene is not expressed in the nucleus; b is the number of cells that, after being stained with hematoxylin, show one red/brown mark in the nucleus, meaning the imprinted gene is present in the nucleus; c is the number of cells that, after being stained with hematoxylin, show two red/brown marks in the nucleus, meaning the imprinted gene in the nucleus is affected by a loss of imprinting; and d is the number of cells that, after being stained with hematoxylin, show more than two red/brown marks in the nucleus, meaning the imprinted gene in the nucleus has a copy number variation.

The hematoxylin-stained marks are selected from but not limited to red marks and brown marks. Staining/marking in other colors is also feasible when calculating the total expressed quantity of an imprinted gene, the expressed quantity of the imprinted gene with a loss of imprinting, and the expressed quantity of the imprinted gene with a copy number variation.

The in-situ hybridization is carried out by way of the probe. The nuclei of cells are stained with hematoxylin for signal amplification. Each nucleus is detected under a 40× or 60× microscope for the presence of the to-be-detected imprinted gene, for any loss of imprinting in the imprinted gene, and for any copy number variation in the imprinted gene. The degree of benignity/malignancy of the tumor sample in question is determined by calculating the total expressed quantity of the imprinted gene, the expressed quantity of the imprinted gene with a loss of imprinting, and the expressed quantity of the imprinted gene with a copy number variation. As the section under detection is merely 10 μm thick, about 20% of the nuclei seen under the microscope are incomplete; in other words, the detection results may be partly false negative.

In one embodiment of the present disclosure, the expressed quantity of the to-be-detected imprinted gene with a loss of imprinting, the expressed quantity of the imprinted gene with a copy number variation, and the total expressed quantity of the imprinted gene are graded into five different grades. The foregoing expressed quantities of each of ten imprinted genes, namely Z1, Z3, Z4, Z5, Z6, Z8, Z10, Z11, Z13, and Z16, are obtained by counting at least 1200 cells in an area of a sample where the corresponding probe is the most clearly expressed, and the expressed quantities obtained are classified according to the five-grade scale.

The expressed quantities of the imprinted genes Z1, Z3, Z11, and Z16 with a loss of imprinting, the expressed quantities of the imprinted genes Z1, Z3, Z11, and Z16 with a copy number variation, and the respective total expressed quantities of the imprinted genes Z1, Z3, Z11, and Z16 are classified into the following five grades:

Grade 0: applicable when any one or at least two of the following conditions are met: the expressed quantity of the imprinted gene Z1, Z3, Z11, or Z16 with a loss of imprinting is less than 10%, the expressed quantity of the imprinted gene Z1, Z3, Z11, or Z16 with a copy number variation is less than 1%, and the total expressed quantity of the imprinted gene Z1, Z3, Z11, or Z16 is less than 20%;

Grade I: applicable when any one or at least two of the following conditions are met: the expressed quantity of the imprinted gene Z1, Z3, Z11, or Z16 with a loss of imprinting is 10%-20%, the expressed quantity of the imprinted gene Z1, Z3, Z11, or Z16 with a copy number variation is 1%-3%, and the total expressed quantity of the imprinted gene Z1, Z3, Z11, or Z16 is 20%-30%;

Grade II: applicable when any one or at least two of the following conditions are met: the expressed quantity of the imprinted gene Z1, Z3, Z11, or Z16 with a loss of imprinting is 20%-25%, the expressed quantity of the imprinted gene Z1, Z3, Z11, or Z16 with a copy number variation is 3%-5%, and the total expressed quantity of the imprinted gene Z1, Z3, Z11, or Z16 is 30%-40%;

Grade III: applicable when any one or at least two of the following conditions are met: the expressed quantity of the imprinted gene Z1, Z3, Z11, or Z16 with a loss of imprinting is 25%-30%, the expressed quantity of the imprinted gene Z1, Z3, Z11, or Z16 with a copy number variation is 5%-7%, and the total expressed quantity of the imprinted gene Z1, Z3, Z11, or Z16 is 40%-50%; and

Grade IV: applicable when any one or at least two of the following conditions are met: the expressed quantity of the imprinted gene Z1, Z3, Z11, or Z16 with a loss of imprinting is greater than 30%, the expressed quantity of the imprinted gene Z1, Z3, Z11, or Z16 with a copy number variation is greater than 7%, and the total expressed quantity of the imprinted gene Z1, Z3, Z11, or Z16 is greater than 50%;

wherein the aforesaid expressed quantities of any one of the imprinted genes Z1, Z3, Z11, and Z16 are independent of those of another.

The expressed quantities of the imprinted genes Z4, Z5, Z6, Z10, and Z13 with a loss of imprinting, the expressed quantities of the imprinted genes Z4, Z5, Z6, Z10, and Z13 with a copy number variation, and the respective total expressed quantities of the imprinted genes Z4, Z5, Z6, Z10, and Z13 are classified into the following five grades:

Grade 0: applicable when any one or at least two of the following conditions are met: the expressed quantity of the imprinted gene Z4, Z5, Z6, Z10, or Z13 with a loss of imprinting is less than 10%, the expressed quantity of the imprinted gene Z4, Z5, Z6, Z10, or Z13 with a copy number variation is less than 1%, and the total expressed quantity of the imprinted gene Z4, Z5, Z6, Z10, or Z13 is less than 15%;

Grade I: applicable when any one or at least two of the following conditions are met: the expressed quantity of the imprinted gene Z4, Z5, Z6, Z10, or Z13 with a loss of imprinting is 10%-15%, the expressed quantity of the imprinted gene Z4, Z5, Z6, Z10, or Z13 with a copy number variation is 1%-2%, and the total expressed quantity of the imprinted gene Z4, Z5, Z6, Z10, or Z13 is 15%-20%;

Grade II: applicable when any one or at least two of the following conditions are met: the expressed quantity of the imprinted gene Z4, Z5, Z6, Z10, or Z13 with a loss of imprinting is 15%-20%, the expressed quantity of the imprinted gene Z4, Z5, Z6, Z10, or Z13 with a copy number variation is 2%-4%, and the total expressed quantity of the imprinted gene Z4, Z5, Z6, Z10, or Z13 is 20%-30%;

Grade III: applicable when any one or at least two of the following conditions are met: the expressed quantity of the imprinted gene Z4, Z5, Z6, Z10, or Z13 with a loss of imprinting is 20%-25%, the expressed quantity of the imprinted gene Z4, Z5, Z6, Z10, or Z13 with a copy number variation is 4%-6%, and the total expressed quantity of the imprinted gene Z4, Z5, Z6, Z10, or Z13 is 30%-40%; and

Grade IV: applicable when any one or at least two of the following conditions are met: the expressed quantity of the imprinted gene Z4, Z5, Z6, Z10, or Z13 with a loss of imprinting is greater than 25%, the expressed quantity of the imprinted gene Z4, Z5, Z6, Z10, or Z13 with a copy number variation is greater than 6%, and the total expressed quantity of the imprinted gene Z4, Z5, Z6, Z10, or Z13 is greater than 40%;

wherein the aforesaid expressed quantities of any one of the imprinted genes Z4, Z5, Z6, Z10, and Z13 are independent of those of another.

The expressed quantity of the imprinted gene Z8 with a loss of imprinting, the expressed quantity of the imprinted gene Z8 with a copy number variation, and the total expressed quantity of the imprinted gene Z8 are classified into the following five grades:

Grade 0: applicable when any one or at least two of the following conditions are met: the expressed quantity of the imprinted gene Z8 with a loss of imprinting is less than 20%, the expressed quantity of the imprinted gene Z8 with a copy number variation is less than 2%, and the total expressed quantity of the imprinted gene Z8 is less than 15%;

Grade I: applicable when any one or at least two of the following conditions are met: the expressed quantity of the imprinted gene Z8 with a loss of imprinting is 20%-25%, the expressed quantity of the imprinted gene Z8 with a copy number variation is 2%-4%, and the total expressed quantity of the imprinted gene Z8 is 15%-20%;

Grade II: applicable when any one or at least two of the following conditions are met: the expressed quantity of the imprinted gene Z8 with a loss of imprinting is 25%-30%, the expressed quantity of the imprinted gene Z8 with a copy number variation is 4%-7%, and the total expressed quantity of the imprinted gene Z8 is 20%-30%;

Grade III: applicable when any one or at least two of the following conditions are met: the expressed quantity of the imprinted gene Z8 with a loss of imprinting is 30%-35%, the expressed quantity of the imprinted gene Z8 with a copy number variation is 7%-10%, and the total expressed quantity of the imprinted gene Z8 is 30%-40%; and

Grade IV: applicable when any one or at least two of the following conditions are met: the expressed quantity of the imprinted gene Z8 with a loss of imprinting is greater than 35%, the expressed quantity of the imprinted gene Z8 with a copy number variation is greater than 10%, and the total expressed quantity of the imprinted gene Z8 is greater than 40%.

In one embodiment of the present disclosure, the degree of benignity/malignancy of a lung tumor is classified as one of the following: benign tumor, potential lung cancer, early-stage lung cancer, intermediate-stage lung cancer, and advanced lung cancer.

In one embodiment of the present disclosure, the degree of benignity/malignancy of a lung tumor is determined as benign tumor when the expressed quantities of each of the imprinted genes Z1, Z3, Z4, Z5, Z6, Z8, Z10, Z11, Z13, and Z16 with, respectively, a loss of imprinting and a copy number variation are both graded as Grade 0; or when the expressed quantity of not more than one of the imprinted genes Z1, Z3, Z4, Z5, Z6, Z8, Z10, Z11, Z13, and Z16 with a loss of imprinting is graded as Grade I, and the expressed quantity of not more than one of the imprinted genes Z1, Z3, Z4, Z5, Z6, Z8, Z10, Z11, Z13, and Z16 with a copy number variation is graded as Grade I;

the degree of benignity/malignancy of the lung tumor is determined as potential lung cancer when the expressed quantities of at least two of the imprinted genes Z1, Z3, Z4, Z5, Z6, Z8, Z10, Z11, Z13, and Z16 with a loss of imprinting are graded as Grade I; or when the expressed quantities of at least two of the imprinted genes Z1, Z3, Z4, Z5, Z6, Z8, Z10, Z11, Z13, and Z16 with a copy number variation are graded as Grade I; or when the expressed quantity of not more than one of the imprinted genes Z1, Z3, Z4, Z5, Z6, Z8, Z10, Z11, Z13, and Z16 with a loss of imprinting is graded as Grade II, and the expressed quantity of not more than one of the imprinted genes Z1, Z3, Z4, Z5, Z6, Z8, Z10, Z11, Z13, and Z16 with a copy number variation is graded as Grade II;

the degree of benignity/malignancy of the lung tumor is determined as early-stage lung cancer when the expressed quantities of at least two of the imprinted genes Z1, Z3, Z4, Z5, Z6, Z8, Z10, Z11, Z13, and Z16 with a loss of imprinting are graded as Grade II; or when the expressed quantities of at least two of the imprinted genes Z1, Z3, Z4, Z5, Z6, Z8, Z10, Z11, Z13, and Z16 with a copy number variation are graded as Grade II; or when the expressed quantity of not more than one of the imprinted genes Z1, Z3, Z4, Z5, Z6, Z8, Z10, Z11, Z13, and Z16 with a loss of imprinting is graded as Grade III, and the expressed quantity of not more than one of the imprinted genes Z1, Z3, Z4, Z5, Z6, Z8, Z10, Z11, Z13, and Z16 with a copy number variation is graded as Grade III;

the degree of benignity/malignancy of the lung tumor is determined as intermediate-stage lung cancer when the expressed quantities of at least two of the imprinted genes Z1, Z3, Z4, Z5, Z6, Z8, Z10, Z11, Z13, and Z16 with a loss of imprinting are graded as Grade III; or when the expressed quantities of at least two of the imprinted genes Z1, Z3, Z4, Z5, Z6, Z8, Z10, Z11, Z13, and Z16 with a copy number variation are graded as Grade III; or when the expressed quantity of not more than one of the imprinted genes Z1, Z3, Z4, Z5, Z6, Z8, Z10, Z11, Z13, and Z16 with a loss of imprinting is graded as Grade IV, and the expressed quantity of not more than one of the imprinted genes Z1, Z3, Z4, Z5, Z6, Z8, Z10, Z11, Z13, and Z16 with a copy number variation is graded as Grade IV; and

the degree of benignity/malignancy of the lung tumor is determined as advanced lung cancer when the expressed quantities of at least two of the imprinted genes Z1, Z3, Z4, Z5, Z6, Z8, Z10, Z11, Z13, and Z16 with a loss of imprinting are graded as Grade IV; or when the expressed quantities of at least two of the imprinted genes Z1, Z3, Z4, Z5, Z6, Z8, Z10, Z11, Z13, and Z16 with a copy number variation are graded as Grade IV.

In one embodiment of the present disclosure, a use of the foregoing model or of the foregoing device in detecting a lung tumor is provided.

In one embodiment of the present disclosure, a use of the foregoing model or of the foregoing device in preparing or making a medicine or instrument for treating lung cancer is provided.

In one embodiment of the present disclosure, the degree of benignity/malignancy of a lung tumor is classified as one of the following: benign tumor, potential lung cancer, early-stage lung cancer, intermediate-stage lung cancer, and advanced lung cancer.

In one embodiment of the present disclosure, the degree of benignity/malignancy of a lung tumor is determined as benign tumor when the expressed quantities of each of the imprinted genes Z1, Z3, Z4, Z5, Z6, Z8, Z10, Z11, Z13, and Z16 with, respectively, a loss of imprinting and a copy number variation are both graded as Grade 0; or when the expressed quantity of not more than one of the imprinted genes Z1, Z3, Z4, Z5, Z6, Z8, Z10, Z11, Z13, and Z16 with a loss of imprinting is graded as Grade I, and the expressed quantity of not more than one of the imprinted genes Z1, Z3, Z4, Z5, Z6, Z8, Z10, Z11, Z13, and Z16 with a copy number variation is graded as Grade I;

the degree of benignity/malignancy of the lung tumor is determined as potential lung cancer when the expressed quantities of at least two of the imprinted genes Z1, Z3, Z4, Z5, Z6, Z8, Z10, Z11, Z13, and Z16 with a loss of imprinting are graded as Grade I; or when the expressed quantities of at least two of the imprinted genes Z1, Z3, Z4, Z5, Z6, Z8, Z10, Z11, Z13, and Z16 with a copy number variation are graded as Grade I; or when the expressed quantity of not more than one of the imprinted genes Z1, Z3, Z4, Z5, Z6, Z8, Z10, Z11, Z13, and Z16 with a loss of imprinting is graded as Grade II, and the expressed quantity of not more than one of the imprinted genes Z1, Z3, Z4, Z5, Z6, Z8, Z10, Z11, Z13, and Z16 with a copy number variation is graded as Grade II;

the degree of benignity/malignancy of the lung tumor is determined as early-stage lung cancer when the expressed quantities of at least two of the imprinted genes Z1, Z3, Z4, Z5, Z6, Z8, Z10, Z11, Z13, and Z16 with a loss of imprinting are graded as Grade II; or when the expressed quantities of at least two of the imprinted genes Z1, Z3, Z4, Z5, Z6, Z8, Z10, Z11, Z13, and Z16 with a copy number variation are graded as Grade II; or when the expressed quantity of not more than one of the imprinted genes Z1, Z3, Z4, Z5, Z6, Z8, Z10, Z11, Z13, and Z16 with a loss of imprinting is graded as Grade III, and the expressed quantity of not more than one of the imprinted genes Z1, Z3, Z4, Z5, Z6, Z8, Z10, Z11, Z13, and Z16 with a copy number variation is graded as Grade III;

the degree of benignity/malignancy of the lung tumor is determined as intermediate-stage lung cancer when the expressed quantities of at least two of the imprinted genes Z1, Z3, Z4, Z5, Z6, Z8, Z10, Z11, Z13, and Z16 with a loss of imprinting are graded as Grade III; or when the expressed quantities of at least two of the imprinted genes Z1, Z3, Z4, Z5, Z6, Z8, Z10, Z11, Z13, and Z16 with a copy number variation are graded as Grade III; or when the expressed quantity of not more than one of the imprinted genes Z1, Z3, Z4, Z5, Z6, Z8, Z10, Z11, Z13, and Z16 with a loss of imprinting is graded as Grade IV, and the expressed quantity of not more than one of the imprinted genes Z1, Z3, Z4, Z5, Z6, Z8, Z10, Z11, Z13, and Z16 with a copy number variation is graded as Grade IV; and

the degree of benignity/malignancy of the lung tumor is determined as advanced lung cancer when the expressed quantities of at least two of the imprinted genes Z1, Z3, Z4, Z5, Z6, Z8, Z10, Z11, Z13, and Z16 with a loss of imprinting are graded as Grade IV; or when the expressed quantities of at least two of the imprinted genes Z1, Z3, Z4, Z5, Z6, Z8, Z10, Z11, Z13, and Z16 with a copy number variation are graded as Grade IV.

Compared with the related art, the embodiments disclosed herein use the foregoing detection model or device to enable intuitive observation of the expression of an imprinted gene in a sample taken from a patient with a lung tumor. By labeling the imprinted gene in situ, changes in the imprinted gene can be objectively, intuitively, and precisely detected at an early stage. Not only that, a quantitative model is provided. Thus, the present disclosure contributes greatly to diagnoses in molecular pathology.

**Brief Description of the Several Views of the Drawings**

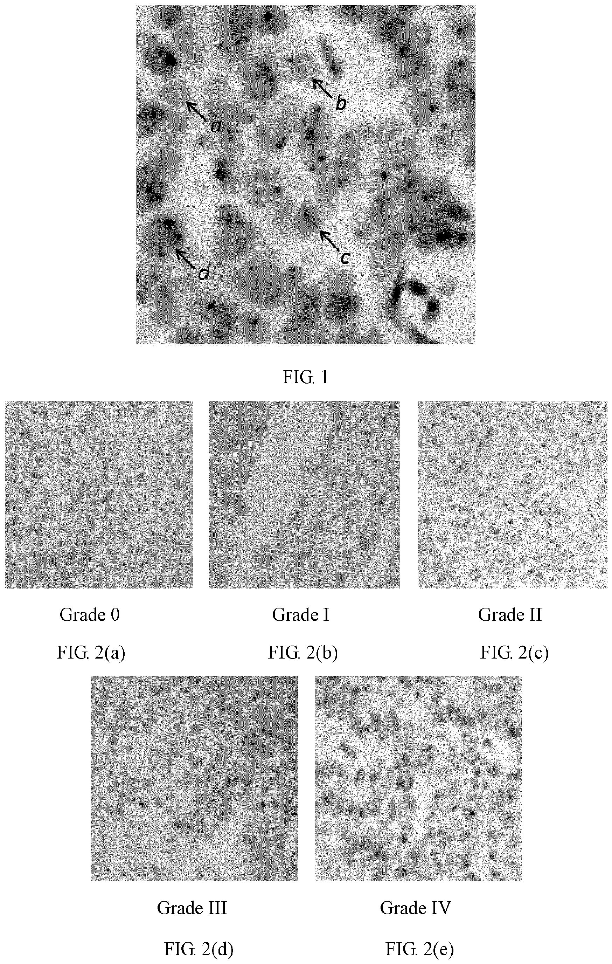

FIG. 1 shows a pathological section containing lung cancer cells whose nuclei are stained with hematoxylin, with a indicating cells that, after being stained with hematoxylin, show no mark in the nucleus, meaning the imprinted gene is not expressed in the nucleus; b indicating cells that, after being stained with hematoxylin, show one red/brown mark in the nucleus, meaning the imprinted gene is present in the nucleus; c indicating cells that, after being stained with hematoxylin, show two red/brown marks in the nucleus, meaning the imprinted gene in the nucleus is affected by a loss of imprinting; and d indicating cells that, after being stained with hematoxylin, show more than two red/brown marks in the nucleus, meaning the imprinted gene in the nucleus has a copy number variation;

FIG. 2(a) shows the expression states of ten genes in a pathological section of a Grade 0 lung tumor, FIG. 2(b) shows the expression states of ten genes in a pathological section of Grade I lung cancer, FIG. 2(c) shows the expression states of ten genes in a pathological section of Grade II lung cancer, FIG. 2(d) shows the expression states of ten genes in a pathological section of Grade III lung cancer, and FIG. 2(e) shows the expression states of ten genes in a pathological section of Grade IV lung cancer;

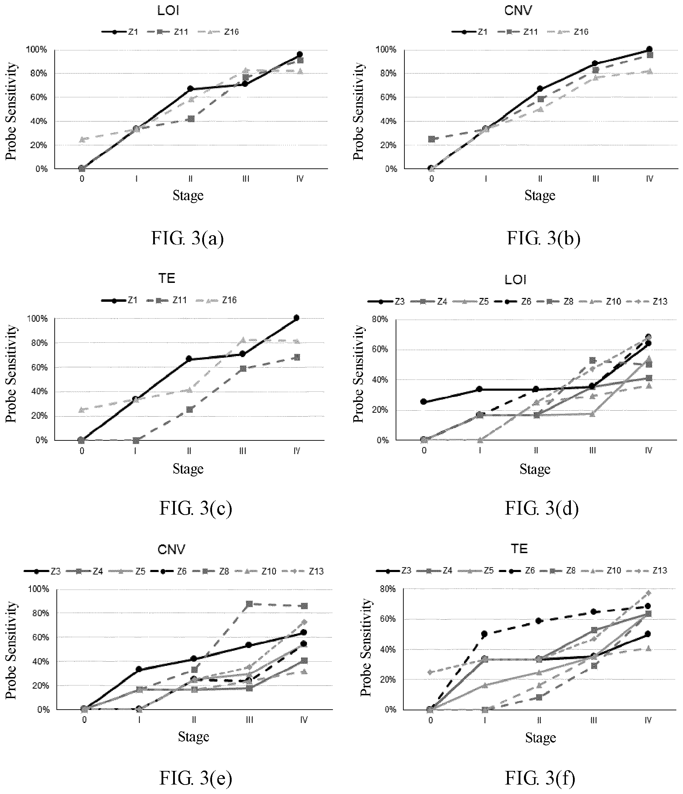

FIG. 3(a) shows the intensities of the imprinted genes Z1, Z11, and Z16 as an indication of their respective expressed quantities corresponding to a loss of imprinting associated with lung cancer; FIG. 3(b) shows the intensities of the imprinted genes Z1, Z11, and Z16 as an indication of their respective expressed quantities corresponding to a copy number variation associated with lung cancer; FIG. 3(c) shows the intensities of the imprinted genes Z1, Z11, and Z16 as an indication of their respective total expressed quantities associated with lung cancer; FIG. 3(d) shows the intensities of the imprinted genes Z3, Z4, Z5, Z6, Z8, Z10, and Z13 as an indication of their respective expressed quantities corresponding to a loss of imprinting associated with lung cancer; FIG. 3(e) shows the intensities of the imprinted genes Z3, Z4, Z5, Z6, Z8, Z10, and Z13 as an indication of their respective expressed quantities corresponding to a copy number variation associated with lung cancer; and FIG. 3(f) shows the intensities of the imprinted genes Z3, Z4, Z5, Z6, Z8, Z10, and Z13 as an indication of their respective total expressed quantities associated with lung cancer, with LOI indicating the expressed quantities of imprinted genes with a loss of imprinting, CNV indicating the expressed quantities of imprinted genes with a copy number variation, and TE indicating the total expressed quantities of imprinted genes;

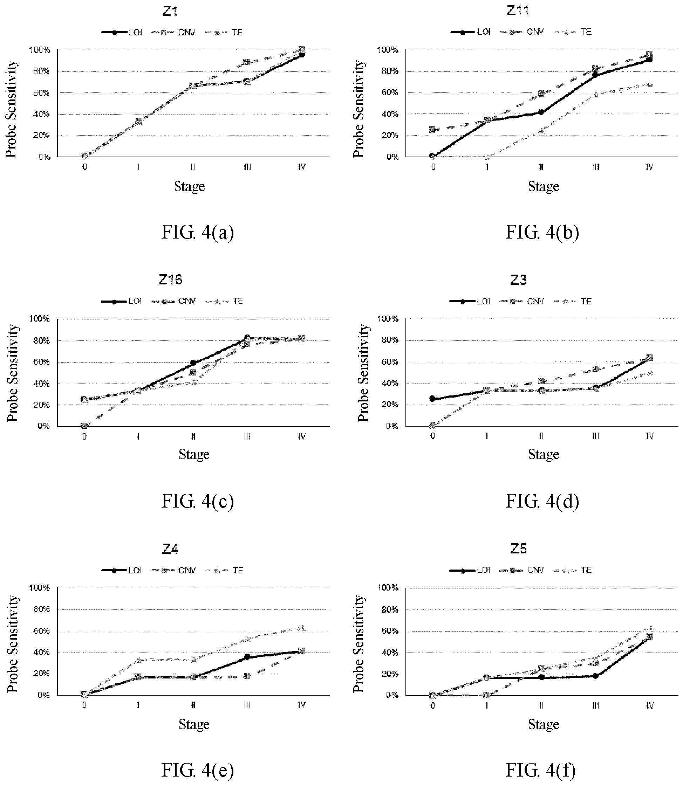

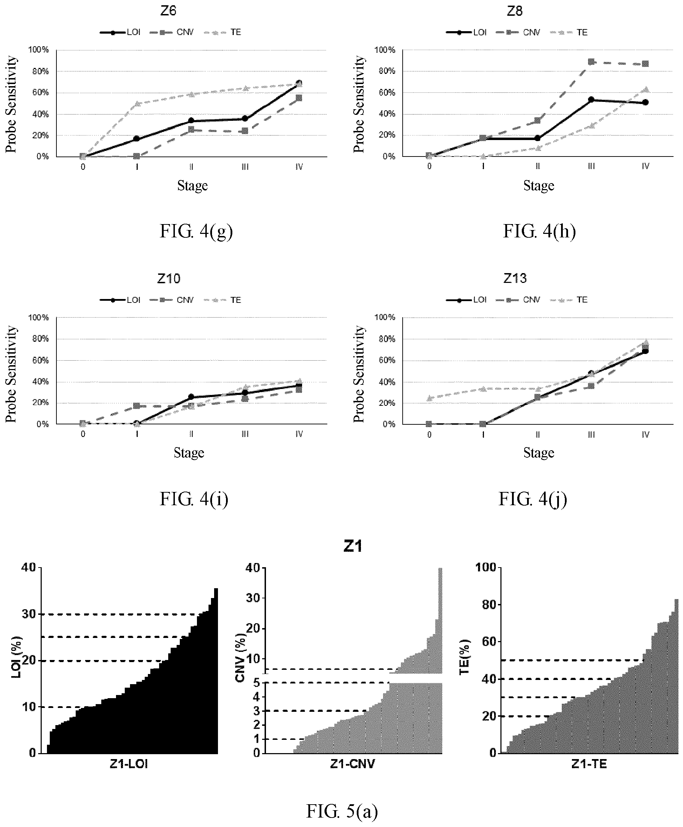

FIG. 4(a) shows the intensities of the imprinted gene Z1 as an indication of its expressed quantities corresponding to a loss of imprinting or a copy number variation or of its total expressed quantities, FIG. 4(b) shows the intensities of the imprinted gene Z11 as an indication of its expressed quantities corresponding to a loss of imprinting or a copy number variation or of its total expressed quantities, FIG. 4(c) shows the intensities of the imprinted gene Z16 as an indication of its expressed quantities corresponding to a loss of imprinting or a copy number variation or of its total expressed quantities, FIG. 4(d) shows the intensities of the imprinted gene Z3 as an indication of its expressed quantities corresponding to a loss of imprinting or a copy number variation or of its total expressed quantities, FIG. 4(e) shows the intensities of the imprinted gene Z4 as an indication of its expressed quantities corresponding to a loss of imprinting or a copy number variation or of its total expressed quantities, FIG. 4(f) shows the intensities of the imprinted gene Z5 as an indication of its expressed quantities corresponding to a loss of imprinting or a copy number variation or of its total expressed quantities, FIG. 4(g) shows the intensities of the imprinted gene Z6 as an indication of its expressed quantities corresponding to a loss of imprinting or a copy number variation or of its total expressed quantities, FIG. 4(h) shows the intensities of the imprinted gene Z8 as an indication of its expressed quantities corresponding to a loss of imprinting or a copy number variation or of its total expressed quantities, FIG. 4(i) shows the intensities of the imprinted gene Z10 as an indication of its expressed quantities corresponding to a loss of imprinting or a copy number variation or of its total expressed quantities, and FIG. 4(j) shows the intensities of the imprinted gene Z13 as an indication of its expressed quantities corresponding to a loss of imprinting or a copy number variation or of its total expressed quantities, with LOI indicating the expressed quantities of an imprinted gene with a loss of imprinting, CNV indicating the expressed quantities of an imprinted gene with a copy number variation, and TE indicating the total expressed quantities of an imprinted gene; and

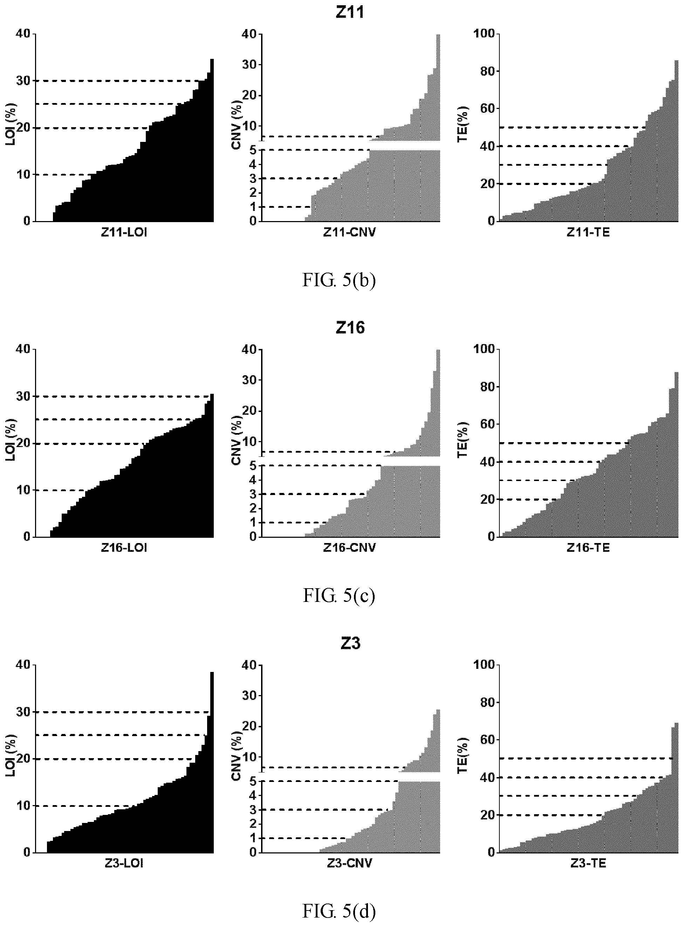

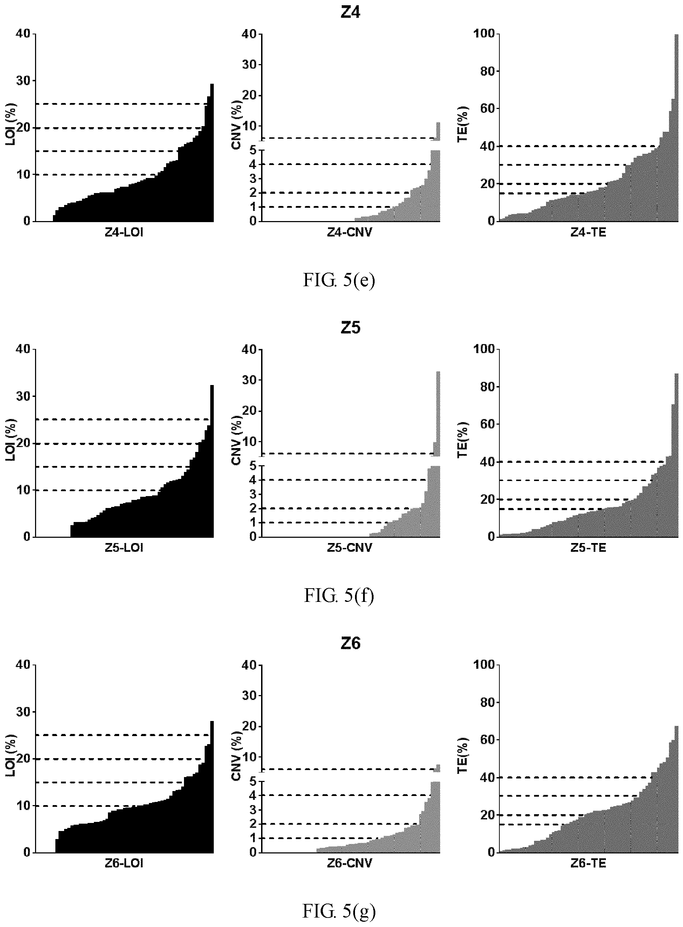

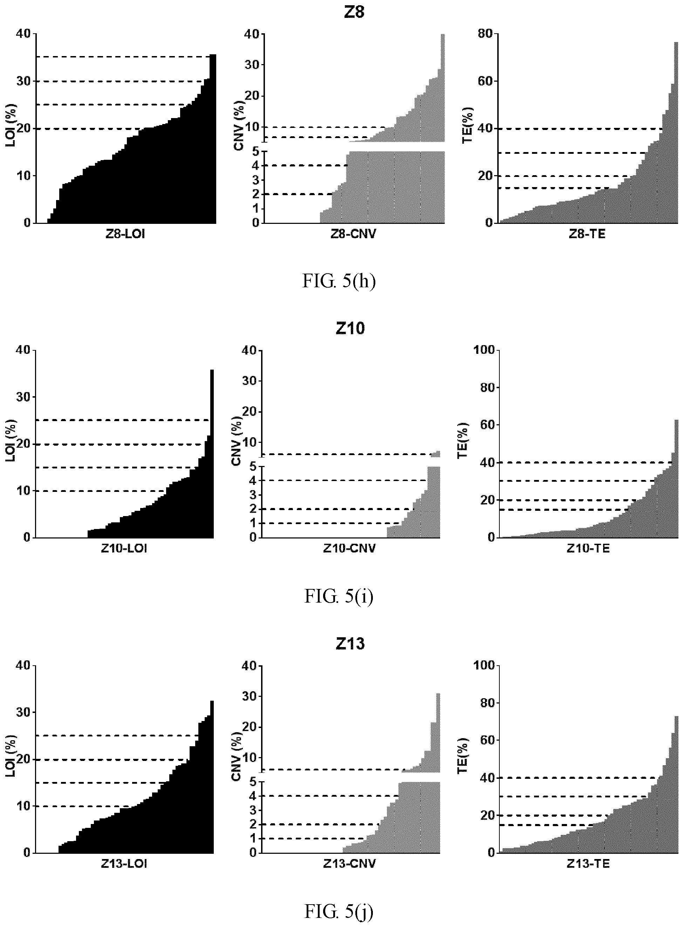

FIG. 5(a) shows the distributions and grading criteria of the loss-of-imprinting-related and copy-number-variation-related expressed quantities of the imprinted gene Z1 in 61 pathological sections of lung cancer, FIG. 5(b) shows the distributions and grading criteria of the loss-of-imprinting-related and copy-number-variation-related expressed quantities of the imprinted gene Z11 in 61 pathological sections of lung cancer, FIG. 5(c) shows the distributions and grading criteria of the loss-of-imprinting-related and copy-number-variation-related expressed quantities of the imprinted gene Z16 in 61 pathological sections of lung cancer, FIG. 5(d) shows the distributions and grading criteria of the loss-of-imprinting-related and copy-number-variation-related expressed quantities of the imprinted gene Z3 in 61 pathological sections of lung cancer, FIG. 5(e) shows the distributions and grading criteria of the loss-of-imprinting-related and copy-number-variation-related expressed quantities of the imprinted gene Z4 in 61 pathological sections of lung cancer, FIG. 5(f) shows the distributions and grading criteria of the loss-of-imprinting-related and copy-number-variation-related expressed quantities of the imprinted gene Z5 in 61 pathological sections of lung cancer, FIG. 5(g) shows the distributions and grading criteria of the loss-of-imprinting-related and copy-number-variation-related expressed quantities of the imprinted gene Z6 in 61 pathological sections of lung cancer, FIG. 5(h) shows the distributions and grading criteria of the loss-of-imprinting-related and copy-number-variation-related expressed quantities of the imprinted gene Z8 in 61 pathological sections of lung cancer, FIG. 5(i) shows the distributions and grading criteria of the loss-of-imprinting-related and copy-number-variation-related expressed quantities of the imprinted gene Z10 in 61 pathological sections of lung cancer, and FIG. 5(j) shows the distributions and grading criteria of the loss-of-imprinting-related and copy-number-variation-related expressed quantities of the imprinted gene Z13 in 61 pathological sections of lung cancer, with LOI indicating the expressed quantities of an imprinted gene with a loss of imprinting, CNV indicating the expressed quantities of an imprinted gene with a copy number variation, and TE indicating the total expressed quantities of an imprinted gene.

## Detailed Description of the Invention

To further explain the technical solution used by the present invention and its effects, certain embodiments are detailed below with reference to the accompanying drawings to expound the technical solution of the invention. The invention, however, is not limited to the embodiments described below.

Genomic imprinting is a gene regulation method in epigenetics and is characterized by methylating an allele from a specific parent such that only one allele of the corresponding gene is expressed while the other allele is in a silenced state. A gene regulated in this way is referred to as an imprinted gene. Loss of imprinting is an epigenetic change in which the silenced allele of an imprinted gene is demethylated and is thus activated and expressed. Numerous studies have shown that loss of imprinting exists widely in all kinds of cancers and takes place earlier than morphological changes in cells and tissue. Loss of imprinting, however, seldom occurs in healthy cells, which is in stark contrast to the case with cancer cells. Therefore, the methylated state of an imprinted gene can serve as a pathological marker and used in conjunction with specific molecular detection techniques to analyze cellular abnormality.

The present disclosure provides a detection model and device that enable intuitive observation of the expression of an imprinted gene with a loss of imprinting in a sample taken from a patient with a lung tumor. By labeling the imprinted gene in situ, changes in the imprinted gene can be objectively, intuitively, and precisely detected at an early stage. Not only that, a quantitative model is provided. Thus, the disclosure contributes greatly to the diagnosis of lung tumors.

The detection device disclosed herein can be used to determine the degree of benignity/malignancy of a patient's lung tumor through a biopsy sample taken from the patient in a minimally invasive or non-invasive manner before the patient is operated on, thus providing a basis for the surgical operation and precise treatment to be performed. This is a revolutionary breakthrough in the diagnosis of lung tumors on a cellular or molecular level.

The present disclosure makes it possible to precisely diagnose the type of a lung tumor, and by detecting a combination of imprinted genes, early definite diagnosis of lung cancer is enormously enhanced. The disclosure is useful especially in early general surveys and cancer patients' postoperative follow-ups, in particular when cancer recurrence is suspected. The disclosure can shorten the time required for diagnosis and thus contributes greatly to saving human lives.

The detection method disclosed herein is different from its immunohistochemical counterparts in that it can reduce false positives and other negative effects. Moreover, the discovery of targeting drugs or methods that work at the loss-of-imprinting-affected sites of lung-tumor-related imprinted genes to silence, delete, or rearrange those genes can be used to guide subsequent treatment and medication.

**Embodiment 1: imprinted gene analysis for lung cancer**

The imprinted gene detection method used in this embodiment includes the following steps:

(1) Lung cancer tissue or cells were obtained and placed in 10% neutral buffered formalin in order to be fixed, lest the RNA degrade. After fixing for 24 hours and embedment in paraffin (FFPE), the embedded tissue or cells were cut into 10 μm-thick sections, which were loaded onto positively charged slides. The slides were then baked in a 40°C oven for at least 3 hours.
(2) The sample processing methods of RNAscope were used to dewax the sections, block the activity of the endogenous peroxidase in the samples, permeabilize the tissue or cells, and expose the RNA molecules.
(3) Probe design: Specific primers, or probes, were designed according to the sequences of the imprinted genes to be detected.

The probes were designed according to the to-be-detected imprinted genes Z1 (Gnas), Z3 (Peg10), Z4 (Igf2r), Z5 (Mest), Z6 (Plagl1), Z8 (Dcn), Z10 (Gatm), Z11 (Grb10), Z13 (Sgce), and Z16 (Snrpn/Snurf). More specifically, a sequence was selected from the intron of each of the aforesaid genes as the corresponding probe. The probes were designed by Advanced Cell Diagnostics.

(4) RNAscope reagent kits were used to perform in-situ hybridization between the probes in step (3) and the test samples.
(5) After staining with hematoxylin for signal amplification, the expression of the imprinted genes was analyzed via microscopic imaging.

The total expressed quantity of each imprinted gene, the expressed quantity of each imprinted gene with a loss of imprinting, and the expressed quantity of each imprinted gene with a copy number variation were calculated by the foregoing model using the following formulas:

$$\text{total expressed quantity of an imprinted gene} = (b+c+d)/(a+b+c+d) \times 100\%;$$

$$\text{expressed quantity of the imprinted gene being normal} = b/(b+c+d) \times 100\%;$$

$$\text{expressed quantity of the imprinted gene with a loss of imprinting (LOI)} = c/(b+c+d) \times 100\%;$$

and

$$\text{expressed quantity of the imprinted gene with a copy number variation (CNV)} = d/(b+c+d) \times 100\%;$$

where a, b, c, and d are shown in FIG. 1, with a being the number of cells that, after being stained with hematoxylin, show no mark in the nucleus, meaning the imprinted gene is not expressed in the nucleus; b being the number of cells that, after being stained with hematoxylin, show one red/brown mark in the nucleus, meaning the imprinted gene is present in the nucleus; c being the number of cells that, after being stained with hematoxylin, show two red/brown marks in the nucleus, meaning the imprinted gene in the nucleus is affected by a loss of imprinting; and d being the number of cells that, after being stained with hematoxylin, show more than two red/brown marks in the nucleus, meaning the imprinted gene in the nucleus has a copy number variation.

As can be seen in the Grade 0 to Grade IV samples in FIG. 2(a) to FIG. 2(e), the proportions of cells with a loss of imprinting (i.e., cells with two signal points in the nucleus) and of cells with a copy number variation (i.e., cells with three or more signal points in the nucleus) gradually increased with the degree of malignancy.

**Embodiment 2: analysis of imprinted genes in biopsy samples obtained through bronchoscopy**

The biopsy samples obtained through bronchoscopy for use in this embodiment were suspicious lesion tissue extracted via a bronchoscope and were fixed with 10% neutral buffered formalin for at least 24 hours. The remaining steps of the detection method were the same as those in embodiment 1.

As can be seen in FIG. 3(a) to FIG. 3(f), each of the genes Z1, Z3, Z4, Z5, Z6, Z8, Z10, Z11, Z13, and Z16 had a different reaction sensitivity to lung cancer from another; in other words, the expression intensities and states of each gene with a loss of imprinting associated with lung cancer were different from those of another.

The sensitivity of each imprinted gene to lung cancer is shown in the corresponding plot in FIG. 4(a) to FIG. 4(j). Referring to FIG. 4(a), loss of imprinting, copy number variation, and an increase of the total expressed quantity of the imprinted gene Z1 began to show in the potentially malignant stage, and the loss of imprinting, the copy number variation, and the total expressed quantity of the imprinted gene Z1 rose rapidly in the early-stage lung cancer stage and kept rising to very high levels as the lung cancer developed. Referring to FIG. 4(b), loss of imprinting and copy number variation of the imprinted gene Z11 began to increase in the potentially malignant stage and reached very high levels as the lung cancer developed to the advanced stage, and the total expressed quantity of the imprinted gene Z11 began to increase in the early-stage lung cancer stage and reached a relatively high level as the lung cancer developed to the advanced stage. Referring to FIG. 4(c), loss of imprinting, copy number variation, and the total expressed quantity of the imprinted gene Z16 began to increase in the potentially malignant stage, reached very high levels in the intermediate-stage lung cancer stage, and remained at their respective levels in the advanced lung cancer stage.

Referring to FIG. 4(d), loss of imprinting of the imprinted gene Z3 increased slowly in the potentially malignant stage through the intermediate-stage lung cancer stage and rose rapidly to a relatively high level in the advanced lung cancer stage, copy number variation of the imprinted gene Z3 began to show in the potentially malignant stage and gradually rose to a relatively high level as the lung cancer developed, and the total expressed quantity of the imprinted gene Z3 began to increase in the potentially malignant stage and rose slowly in the early-stage lung cancer stage through the advanced lung cancer stage. Referring to FIG. 4(e), loss of imprinting and an increase of the total expressed quantity of the imprinted gene Z4 began to show in the potentially malignant stage, and the loss of imprinting and the total expressed quantity of the imprinted gene Z4 remained stable in the early-stage lung cancer stage and rose again in the intermediate-stage lung cancer stage through the advanced lung cancer stage; and copy number variation of the imprinted gene Z4 began to show in the potentially malignant stage, barely rose in the early-stage lung cancer stage through the intermediate-stage lung cancer stage, went up again in the advanced lung cancer stage, but still failed to show a high level of sensitivity. Referring to FIG. 4(f), loss of imprinting and an increase of the total expressed quantity of the imprinted gene Z5 began to show in the potentially malignant stage, and the loss of imprinting and the total expressed quantity of the imprinted gene Z5 remained stable in the early-stage lung cancer stage and rose again to relatively high levels in the intermediate-stage lung cancer stage through the advanced lung cancer stage; and copy number variation of the imprinted gene Z5 began to show in the early-stage lung cancer stage, barely rose in the intermediate-stage lung cancer stage, and went up again in the advanced lung cancer stage. Referring to FIG. 4(g), loss of imprinting of the imprinted gene Z6 increased gradually in the potentially malignant stage through the early-stage lung cancer stage, remained stable in the intermediate-stage lung cancer stage, and rose rapidly to a relatively high level in the advanced lung cancer stage; copy number variation of the imprinted gene Z6 began to show in the early-stage lung cancer stage, remained stable in the intermediate-stage lung cancer stage, and rose rapidly to a relatively high level in the advanced lung cancer stage; and the total expressed quantity of the imprinted gene Z6 increased rapidly to a relatively high level in the potentially malignant stage and kept rising slowly in the early-stage lung cancer stage through the advanced lung cancer stage. Referring to FIG. 4(h), loss of imprinting of the imprinted gene Z8 began to show in the potentially malignant stage, barely rose in the early-stage lung cancer stage, went up rapidly to a relatively high level in the intermediate-stage lung cancer stage, and remained stable in the advanced lung cancer stage; copy number variation of the imprinted gene Z8 began to show in the potentially malignant stage, rose rapidly to a very high level in the early-stage lung cancer stage through the intermediate-stage lung cancer stage, and remained stable in the advanced lung cancer stage; and the total expressed quantity of the imprinted gene Z8 began to increase in the early-stage lung cancer stage and rose gradually to a relatively high level in the intermediate-stage lung cancer stage through the advanced lung cancer stage. Referring to FIG. 4(i), loss of imprinting and an increase of the total expressed quantity of the imprinted gene Z10 began to show in the early-stage lung cancer stage, and the loss of imprinting and the total expressed quantity of the imprinted gene Z10 rose slowly as the lung cancer developed but still failed to show a high level of sensitivity in the advanced lung cancer stage; and copy number variation of the imprinted gene Z10 began to show in the potentially malignant stage, rose slowly as the lung cancer developed, but still failed to show a high level of sensitivity in the advanced lung cancer stage. Referring to FIG. 4(j), loss of imprinting and copy number variation of the imprinted gene Z13 began to show in the early-stage lung cancer stage and kept rising to relatively high levels in the intermediate-stage lung cancer stage through the advanced lung cancer stage; and the total expressed quantity of the imprinted gene Z13 began to increase in the potentially malignant stage, barely rose in the early-stage lung cancer stage, and went up to a relatively high level in the intermediate-stage lung cancer stage through the advanced lung cancer stage.

**Embodiment 3: analysis of imprinted genes in 61 lung tumor samples**

61 tissue samples (10 $\mu$m thick), including biopsy samples obtained through bronchoscopy, were obtained from lung cancer patients. The detection method used was the same as that in embodiment 1.

FIG. 5(a) to FIG. 5(j) show the distributions of the expressed quantities (arranged in order of increasing percentage) corresponding respectively to a loss of imprinting and a copy number variation as detected by the ten probes used in the lung tumor tissue samples. Based on the trends of distribution corresponding respectively to the different probes, we calculated the dashed-line grading criteria in each plot so as to classify the expressed quantities detected by each probe (namely the expressed quantity corresponding to a loss of imprinting, the expressed quantity corresponding to a copy number variation, and the total expressed quantity) into five grades in order of increasing magnitude.

More specifically, the grading was carried out as follows:

Regarding the imprinted gene Z1, referring to FIG. 5(a), Grade 0 was given when any one or at least two of the following conditions were met: the expressed quantity of the imprinted gene with a loss of imprinting is less than 10%, the expressed quantity of the imprinted gene with a copy number variation is less than 1%, and the total expressed quantity of the imprinted gene is less than 20%; Grade I was given when any one or at least two of the following conditions were met: the expressed quantity of the imprinted gene with a loss of imprinting is 10%-20%, the expressed quantity of the imprinted gene with a copy number variation is 1%-3%, and the total expressed quantity of the imprinted gene is 20%-30%; Grade II was given when any one or at least two of the following conditions were met: the expressed quantity of the imprinted gene with a loss of imprinting is 20%-25%, the expressed quantity of the imprinted gene with a copy number variation is 3%-5%, and the total expressed quantity of the imprinted gene is 30%-40%; Grade III was given when any one or at least two of the following conditions were met: the expressed quantity of the imprinted gene with a loss of imprinting is 25%-30%, the expressed quantity of the imprinted gene with a copy number variation is 5%-7%, and the total expressed quantity of the imprinted gene is 40%-50%; and Grade IV was given when any one or at least two of the following conditions were met: the expressed quantity of the imprinted gene with a loss of imprinting is greater than 30%, the expressed quantity of the imprinted gene with a copy number variation is greater than 7%, and the total expressed quantity of the imprinted gene is greater than 50%.

Regarding the imprinted gene Z11, referring to FIG. 5(b), Grade 0 was given when any one or at least two of the following conditions were met: the expressed quantity of the imprinted gene with a loss of imprinting is less than 10%, the expressed quantity of the imprinted gene with a copy number variation is less than 1%, and the total expressed quantity of the imprinted gene is less than 20%; Grade I was given when any one or at least two of the following conditions were met: the expressed quantity of the imprinted gene with a loss of imprinting is 10%-20%, the expressed quantity of the imprinted gene with a copy number variation is 1%-3%, and the total expressed quantity of the imprinted gene is 20%-30%; Grade II was given when any one or at least two of the following conditions were met: the expressed quantity of the imprinted gene with a loss of imprinting is 20%-25%, the expressed quantity of the imprinted gene with a copy number variation is 3%-5%, and the total expressed quantity of the imprinted gene is 30%-40%; Grade III was given when any one or at least two of the following conditions were met: the expressed quantity of the imprinted gene with a loss of imprinting is 25%-30%, the expressed quantity of the imprinted gene with a copy number variation is 5%-7%, and the total expressed quantity of the imprinted gene is 40%-50%; and Grade IV was given when any one or at least two of the following conditions were met: the expressed quantity of the imprinted gene with a loss of imprinting is greater than 30%, the expressed quantity of the imprinted gene with a copy number variation is greater than 7%, and the total expressed quantity of the imprinted gene is greater than 50%.

Regarding the imprinted gene Z16, referring to FIG. 5(c), Grade 0 was given when any one or at least two of the following conditions were met: the expressed quantity of the imprinted gene with a loss of imprinting is less than 10%, the expressed quantity of the imprinted gene with a copy number variation is less than 1%, and the total expressed quantity of the imprinted gene is less than 20%; Grade I was given when any one or at least two of the following conditions were met: the expressed quantity of the imprinted gene with a loss of imprinting is 10%-20%, the expressed quantity of the imprinted gene with a copy number variation is 1%-3%, and the total expressed quantity of the imprinted gene is 20%-30%; Grade II was given when any one or at least two of the following conditions were met: the expressed quantity of the imprinted gene with a loss of imprinting is 20%-25%, the expressed quantity of the imprinted gene with a copy number variation is 3%-5%, and the total expressed quantity of the imprinted gene is 30%-40%; Grade III was given when any one or at least two of the following conditions were met: the expressed quantity of the imprinted gene with a loss of imprinting is 25%-30%, the expressed quantity of the imprinted gene with a copy number variation is 5%-7%, and the total expressed quantity of the imprinted gene is 40%-50%; and Grade IV was given when any one or at least two of the following conditions were met: the expressed quantity of the imprinted gene with a loss of imprinting is greater than 30%, the expressed quantity of the imprinted gene with a copy number variation is greater than 7%, and the total expressed quantity of the imprinted gene is greater than 50%.

Regarding the imprinted gene Z3, referring to FIG. 5(d), Grade 0 was given when any one or at least two of the following conditions were met: the expressed quantity of the imprinted gene with a loss of imprinting is less than 10%, the expressed quantity of the imprinted gene with a copy number variation is less than 1%, and the total expressed quantity of the imprinted gene is less than 20%; Grade I was given when any one or at least two of the following conditions were met: the expressed quantity of the imprinted gene with a loss of imprinting is 10%-20%, the expressed quantity of the imprinted gene with a copy number variation is 1%-3%, and the total expressed quantity of the imprinted gene is 20%-30%; Grade II was given when any one or at least two of the following conditions were met: the expressed quantity of the imprinted gene with a loss of imprinting is 20%-25%, the expressed quantity of the imprinted gene with a copy number variation is 3%-5%, and the total expressed quantity of the imprinted gene is 30%-40%; Grade III was given when any one or at least two of the following conditions were met: the expressed quantity of the imprinted gene with a loss of imprinting is 25%-30%, the expressed quantity of the imprinted gene with a copy number variation is 5%-7%, and the total expressed quantity of the imprinted gene is 40%-50%; and Grade IV was given when any one or at least two of the following conditions were met: the expressed quantity of the imprinted gene with a loss of imprinting is greater than 30%, the expressed quantity of the imprinted gene with a copy number variation is greater than 7%, and the total expressed quantity of the imprinted gene is greater than 50%.

Regarding the imprinted gene Z4, referring to FIG. 5(e), Grade 0 was given when any one or at least two of the following conditions were met: the expressed quantity of the imprinted gene with a loss of imprinting is less than 10%, the expressed quantity of the imprinted gene with a copy number variation is less than 1%, and the total expressed quantity of the imprinted gene is less than 15%; Grade I was given when any one or at least two of the following conditions were met: the expressed quantity of the imprinted gene with a loss of imprinting is 10%-15%, the expressed quantity of the imprinted gene with a copy number variation is 1%-2%, and the total expressed quantity of the imprinted gene is 15%-20%; Grade II was given when any one or at least two of the following conditions were met: the expressed quantity of the imprinted gene with a loss of imprinting is 15%-20%, the expressed quantity of the imprinted gene with a copy number variation is 2%-4%, and the total expressed quantity of the imprinted gene is 20%-30%; Grade III was given when any one or at least two of the following conditions were met: the expressed quantity of the imprinted gene with a loss of imprinting is 20%-25%, the expressed quantity of the imprinted gene with a copy number variation is 4%-6%, and the total expressed quantity of the imprinted gene is 30%-40%; and Grade IV was given when any one or at least two of the following conditions were met: the expressed quantity of the imprinted gene with a loss of imprinting is greater than 25%, the expressed quantity of the imprinted gene with a copy number variation is greater than 6%, and the total expressed quantity of the imprinted gene is greater than 40%.

Regarding the imprinted gene Z5, referring to FIG. 5(f), Grade 0 was given when any one or at least two of the following conditions were met: the expressed quantity of the imprinted gene with a loss of imprinting is less than 10%, the expressed quantity of the imprinted gene with a copy number variation is less than 1%, and the total expressed quantity of the imprinted gene is less than 15%; Grade I was given when any one or at least two of the following conditions were met: the expressed quantity of the imprinted gene with a loss of imprinting is 10%-15%, the expressed quantity of the imprinted gene with a copy number variation is 1%-2%, and the total expressed quantity of the imprinted gene is 15%-20%; Grade II was given when any one or at least two of the following conditions were met: the expressed quantity of the imprinted gene with a loss of imprinting is 15%-20%, the expressed quantity of the imprinted gene with a copy number variation is 2%-4%, and the total expressed quantity of the imprinted gene is 20%-30%; Grade III was given when any one or at least two of the following conditions were met: the expressed quantity of the imprinted gene with a loss of imprinting is 20%-25%, the expressed quantity of the imprinted gene with a copy number variation is 4%-6%, and the total expressed quantity of the imprinted gene is 30%-40%; and Grade IV was given when any one or at least two of the following conditions were met: the expressed quantity of the imprinted gene with a loss of imprinting is greater than 25%, the expressed quantity of the imprinted gene with a copy number variation is greater than 6%, and the total expressed quantity of the imprinted gene is greater than 40%.

Regarding the imprinted gene Z6, referring to FIG. 5(g), Grade 0 was given when any one or at least two of the following conditions were met: the expressed quantity of the imprinted gene with a loss of imprinting is less than 10%, the expressed quantity of the imprinted gene with a copy number variation is less than 1%, and the total expressed quantity of the imprinted gene is less than 15%; Grade I was given when any one or at least two of the following conditions were met: the expressed quantity of the imprinted gene with a loss of imprinting is 10%-15%, the expressed quantity of the imprinted gene with a copy number variation is 1%-2%, and the total expressed quantity of the imprinted gene is 15%-20%; Grade II was given when any one or at least two of the following conditions were met: the expressed quantity of the imprinted gene with a loss of imprinting is 15%-20%, the expressed quantity of the imprinted gene with a copy number variation is 2%-4%, and the total expressed quantity of the imprinted gene is 20%-30%; Grade III was given when any one or at least two of the following conditions were met: the expressed quantity of the imprinted gene with a loss of imprinting is 20%-25%, the expressed quantity of the imprinted gene with a copy number variation is 4%-6%, and the total expressed quantity of the imprinted gene is 30%-40%; and Grade IV was given when any one or at least two of the following conditions were met: the expressed quantity of the imprinted gene with a loss of imprinting is greater than 25%, the expressed quantity of the imprinted gene with a copy number variation is greater than 6%, and the total expressed quantity of the imprinted gene is greater than 40%.

Regarding the imprinted gene Z8, referring to FIG. 5(h), Grade 0 was given when any one or at least two of the following conditions were met: the expressed quantity of the imprinted gene with a loss of imprinting is less than 20%, the expressed quantity of the imprinted gene with a copy number variation is less than 2%, and the total expressed quantity of the imprinted gene is less than 15%; Grade I was given when any one or at least two of the following conditions were met: the expressed quantity of the imprinted gene with a loss of imprinting is 20%-25%, the expressed quantity of the imprinted gene with a copy number variation is 2%-4%, and the total expressed quantity of the imprinted gene is 15%-20%; Grade II was given when any one or at least two of the following conditions were met: the expressed quantity of the imprinted gene with a loss of imprinting is 25%-30%, the expressed quantity of the imprinted gene with a copy number variation is 4%-7%, and the total expressed quantity of the imprinted gene is 20%-30%; Grade III was given when any one or at least two of the following conditions were met: the expressed quantity of the imprinted gene with a loss of imprinting is 30%-35%, the expressed quantity of the imprinted gene with a copy number variation is 7%-10%, and the total expressed quantity of the imprinted gene is 30%-40%; and Grade IV was given when any one or at least two of the following conditions were met: the expressed quantity of the imprinted gene with a loss of imprinting is greater than 35%, the expressed quantity of the imprinted gene with a copy number variation is greater than 10%, and the total expressed quantity of the imprinted gene is greater than 40%.

Regarding the imprinted gene Z10, referring to FIG. 5(i), Grade 0 was given when any one or at least two of the following conditions were met: the expressed quantity of the imprinted gene with a loss of imprinting is less than 10%, the expressed quantity of the imprinted gene with a copy number variation is less than 1%, and the total expressed quantity of the imprinted gene is less than 15%; Grade I was given when any one or at least two of the following conditions were met: the expressed quantity of the imprinted gene with a loss of imprinting is 10%-15%, the expressed quantity of the imprinted gene with a copy number variation is 1%-2%, and the total expressed quantity of the imprinted gene is 15%-20%; Grade II was given when any one or at least two of the following conditions were met: the expressed quantity of the imprinted gene with a loss of imprinting is 15%-20%, the expressed quantity of the imprinted gene with a copy number variation is 2%-4%, and the total expressed quantity of the imprinted gene is 20%-30%; Grade III was given when any one or at least two of the following conditions were met: the expressed quantity of the imprinted gene with a loss of imprinting is 20%-25%, the expressed quantity of the imprinted gene with a copy number variation is 4%-6%, and the total expressed quantity of the imprinted gene is 30%-40%; and Grade IV was given when any one or at least two of the following conditions were met: the expressed quantity of the imprinted gene with a loss of imprinting is greater than 25%, the expressed quantity of the imprinted gene with a copy number variation is greater than 6%, and the total expressed quantity of the imprinted gene is greater than 40%.

Regarding the imprinted gene Z13, referring to FIG. 5(j), Grade 0 was given when any one or at least two of the following conditions were met: the expressed quantity of the imprinted gene with a loss of imprinting is less than 10%, the expressed quantity of the imprinted gene with a copy number variation is less than 1%, and the total expressed quantity of the imprinted gene is less than 15%; Grade I was given when any one or at least two of the following conditions were met: the expressed quantity of the imprinted gene with a loss of imprinting is 10%-15%, the expressed quantity of the imprinted gene with a copy number variation is 1%-2%, and the total expressed quantity of the imprinted gene is 15%-20%; Grade II was given when any one or at least two of the following conditions were met: the expressed quantity of the imprinted gene with a loss of imprinting is 15%-20%, the expressed quantity of the imprinted gene with a copy number variation is 2%-4%, and the total expressed quantity of the imprinted gene is 20%-30%; Grade III was given when any one or at least two of the following conditions were met: the expressed quantity of the imprinted gene with a loss of imprinting is 20%-25%, the expressed quantity of the imprinted gene with a copy number variation is 4%-6%, and the total expressed quantity of the imprinted gene is 30%-40%; and Grade IV was given when any one or at least two of the following conditions were met: the expressed quantity of the imprinted gene with a loss of imprinting is greater than 25%, the expressed quantity of the imprinted gene with a copy number variation is greater than 6%, and the total expressed quantity of the imprinted gene is greater than 40%.

An integrated analysis of the 61 lung tumor samples led to the following classification:

The degree of benignity/malignancy of a lung tumor can be classified as one of the following: benign tumor, potential lung cancer, early-stage lung cancer, intermediate-stage lung cancer, and advanced lung cancer, as detailed below:

the degree of benignity/malignancy of a lung tumor is determined as benign tumor when the expressed quantities of each of the imprinted genes Z1, Z3, Z4, Z5, Z6, Z8, Z10, Z11, Z13, and Z16 with, respectively, a loss of imprinting and a copy number variation are both graded as Grade 0; or when the expressed quantity of not more than one of the imprinted genes Z1, Z3, Z4, Z5, Z6, Z8, Z10, Z11, Z13, and Z16 with a loss of imprinting is graded as Grade I, and the expressed quantity of not more than one of the imprinted genes Z1, Z3, Z4, Z5, Z6, Z8, Z10, Z11, Z13, and Z16 with a copy number variation is graded as Grade I;

the degree of benignity/malignancy of the lung tumor is determined as potential lung cancer when the expressed quantities of at least two of the imprinted genes Z1, Z3, Z4, Z5, Z6, Z8, Z10, Z11, Z13, and Z16 with a loss of imprinting are graded as Grade I; or when the expressed quantities of at least two of the imprinted genes Z1, Z3, Z4, Z5, Z6, Z8, Z10, Z11, Z13, and Z16 with a copy number variation are graded as Grade I; or when the expressed quantity of not more than one of the imprinted genes Z1, Z3, Z4, Z5, Z6, Z8, Z10, Z11, Z13, and Z16 with a loss of imprinting is graded as Grade II, and the expressed quantity of not more than one of the imprinted genes Z1, Z3, Z4, Z5, Z6, Z8, Z10, Z11, Z13, and Z16 with a copy number variation is graded as Grade II;

the degree of benignity/malignancy of the lung tumor is determined as early-stage lung cancer when the expressed quantities of at least two of the imprinted genes Z1, Z3, Z4, Z5, Z6, Z8, Z10, Z11, Z13, and Z16 with a loss of imprinting are graded as Grade II; or when the expressed quantities of at least two of the imprinted genes Z1, Z3, Z4, Z5, Z6, Z8, Z10, Z11, Z13, and Z16 with a copy number variation are graded as Grade II; or when the expressed quantity of not more than one of the imprinted genes Z1, Z3, Z4, Z5, Z6, Z8, Z10, Z11, Z13, and Z16 with a loss of imprinting is graded as Grade III, and the expressed quantity of not more than one of the imprinted genes Z1, Z3, Z4, Z5, Z6, Z8, Z10, Z11, Z13, and Z16 with a copy number variation is graded as Grade III;

the degree of benignity/malignancy of the lung tumor is determined as intermediate-stage lung cancer when the expressed quantities of at least two of the imprinted genes Z1, Z3, Z4, Z5, Z6, Z8, Z10, Z11, Z13, and Z16 with a loss of imprinting are graded as Grade III; or when the expressed quantities of at least two of the imprinted genes Z1, Z3, Z4, Z5, Z6, Z8, Z10, Z11, Z13, and Z16 with a copy number variation are graded as Grade III; or when the expressed quantity of not more than one of the imprinted genes Z1, Z3, Z4, Z5, Z6, Z8, Z10, Z11, Z13, and Z16 with a loss of imprinting is graded as Grade IV, and the expressed quantity of not more than one of the imprinted genes Z1, Z3, Z4, Z5, Z6, Z8, Z10, Z11, Z13, and Z16 with a copy number variation is graded as Grade IV; and

the degree of benignity/malignancy of the lung tumor is determined as advanced lung cancer when the expressed quantities of at least two of the imprinted genes Z1, Z3, Z4, Z5, Z6, Z8, Z10, Z11, Z13, and Z16 with a loss of imprinting are graded as Grade IV; or when the expressed quantities of at least two of the imprinted genes Z1, Z3, Z4, Z5, Z6, Z8, Z10, Z11, Z13, and Z16 with a copy number variation are graded as Grade IV.

In summary of the above, the detection model and system of the present invention enable intuitive observation of the expression of an imprinted gene with a loss of imprinting in a sample taken from a patient with a lung tumor. By labeling the imprinted gene in situ, changes in the imprinted gene can be objectively, intuitively, and precisely detected at an early stage. Not only that, a quantitative model is provided. Thus, the invention contributes greatly to the diagnosis of lung tumors.

The applicant would like to point out that while the method of the present invention has been described in detail by way of the foregoing embodiments, the invention is not limited to the method detailed above; in other words, implementation of the invention does not necessarily depend on the method detailed above. As would be understood by a person skilled in the art, any improvement made to the invention, any equivalent substitution of, and the addition of any auxiliary ingredient into, the raw materials used in the product of the invention, and any specific method chosen to implement the invention shall fall within the scope of the present disclosure and of the invention patent protection sought by the applicant.

**Claims**

1. An imprinted gene grading model applicable to a lung tumor, the model being configured to calculate changes in the following expressed quantities of an imprinted gene in the lung tumor and grade an expression state of the imprinted gene accordingly: an expressed quantity of the imprinted gene with a loss of imprinting, an expressed quantity of the imprinted gene with a copy number variation, and a total expressed quantity of the imprinted gene; wherein the imprinted gene is any one, or a combination of at least two, of Z1, Z11, and Z16, with the imprinted gene Z1 being Gnas, the imprinted gene Z11 being Grb10, and the imprinted gene Z16 being Snrpn/Snurf.

2. The model of claim 1, wherein the model calculates the expressed quantities of any one of the imprinted genes Z1, Z11, and Z16, preferably the imprinted gene Z1 or Z16, and more preferably the imprinted gene Z1.

3. The model of claim 1 or 2, wherein the model calculates the expressed quantities of a combination of any two of the imprinted genes Z1, Z11, and Z16, preferably a combination of the imprinted genes Z1 and Z16 or a combination of the imprinted genes Z1 and Z11.

4. The model of any of claims 1-3, wherein the imprinted gene further comprises any one, or a combination of at least two, of Z3, Z4, Z5, Z6, Z8, Z10, and Z13, with the imprinted gene Z3 being Peg10, the imprinted gene Z4 being Igf2r, the imprinted gene Z5 being Mest, the imprinted gene Z6 being Plagl1, the imprinted gene Z8 being Dcn, the imprinted gene Z10 being Gatm, and the imprinted gene Z13 being Sgce.

5. The model of any of claims 1-4, wherein the model calculates the expressed quantities of a combination of the ten imprinted genes Z1, Z3, Z4, Z5, Z6, Z8, Z10, Z11, Z13, and Z16.

6. The model of any of claims 1-5, wherein the total expressed quantity of the imprinted gene, the expressed quantity of the imprinted gene with a loss of imprinting, and the expressed quantity of the imprinted gene with a copy number variation are calculated by the following formulas:

$$\text{the total expressed quantity of the imprinted gene} = (b+c+d)/(a+b+c+d)\times 100\%;$$

$$\text{an expressed quantity of the imprinted gene being normal} = b/(b+c+d)\times 100\%;$$

$$\text{the expressed quantity of the imprinted gene with a loss of imprinting (LOI)} = c/(b+c+d)\times 100\%;$$

and

$$\text{the expressed quantity of the imprinted gene with a copy number variation (CNV)} = d/(b+c+d)\times 100\%;$$

where a is the number of cells that, after being stained with hematoxylin, show no mark in the nucleus of each said cell, meaning the imprinted gene is not expressed in the nucleus of each said cell; b is the number of cells that, after being stained with hematoxylin, show one red/brown mark in the nucleus of each said cell, meaning the imprinted gene is present in the nucleus of each said cell; c is the number of cells that, after being stained with hematoxylin, show two red/brown marks in the nucleus of each said cell, meaning the imprinted gene in the nucleus of each said cell is affected by a loss of imprinting; and d is the number of cells that, after being stained with hematoxylin, show more than two red/brown marks in the nucleus of each said cell, meaning the imprinted gene in the nucleus of each said cell has a copy number variation.

7. The model of any of claims 1-6, wherein the expressed quantity of the imprinted gene with a loss of imprinting, the expressed quantity of the imprinted gene with a copy number variation, and the total expressed quantity of the imprinted gene are graded into five different grades.

8. The model of claim 7, wherein the expressed quantity of each of the ten imprinted genes Z1, Z3, Z4, Z5, Z6, Z8, Z10, Z11, Z13, and Z16 with a loss of imprinting, the expressed quantity of each of the ten imprinted genes with a copy number variation, and the total expressed quantity of each of the ten imprinted genes are graded as follows: the expressed quantity of each of the imprinted genes Z1, Z3, Z11, and Z16 with a loss of imprinting, the expressed quantity of each of the imprinted genes Z1, Z3, Z11, and Z16 with a copy number variation, and the total expressed quantity of each of the imprinted genes Z1, Z3, Z11, and Z16 are graded into the following five grades:

Grade 0: applicable when any one or at least two of the following conditions are met: the expressed quantity of one of the imprinted genes Z1, Z3, Z11, and Z16 with a loss of imprinting is less than 10%, the expressed quantity of one of the imprinted genes Z1, Z3, Z11, and Z16 with a copy number variation is less than 1%, and the total expressed quantity of one of the imprinted genes Z1, Z3, Z11, and Z16 is less than 20%;

Grade I: applicable when any one or at least two of the following conditions are met: the expressed quantity of one of the imprinted genes Z1, Z3, Z11, and Z16 with a loss of imprinting is 10%-20%, the expressed quantity of one of the imprinted genes Z1, Z3, Z11, and Z16 with a copy number variation is 1%-3%, and the total expressed quantity of one of the imprinted genes Z1, Z3, Z11, and Z16 is 20%-30%;

Grade II: applicable when any one or at least two of the following conditions are met: the expressed quantity of one of the imprinted genes Z1, Z3, Z11, and Z16 with a loss of imprinting is 20%-25%, the expressed quantity of one of the imprinted genes Z1, Z3, Z11, and Z16 with a copy number variation is 3%-5%, and the total expressed quantity of one of the imprinted genes Z1, Z3, Z11, and Z16 is 30%-40%;

Grade III: applicable when any one or at least two of the following conditions are met: the expressed quantity of one of the imprinted genes Z1, Z3, Z11, and Z16 with a loss of imprinting is 25%-30%, the expressed quantity of one of the imprinted genes Z1, Z3, Z11, and Z16 with a copy number variation is 5%-7%, and the total expressed quantity of one of the imprinted genes Z1, Z3, Z11, and Z16 is 40%-50%; and

Grade IV: applicable when any one or at least two of the following conditions are met: the expressed quantity of one of the imprinted genes Z1, Z3, Z11, and Z16 with a loss of imprinting is greater than 30%, the expressed quantity of one of the imprinted genes Z1, Z3, Z11, and Z16 with a copy number variation is greater than 7%, and the total expressed quantity of one of the imprinted genes Z1, Z3, Z11, and Z16 is greater than 50%;

the expressed quantity of each of the imprinted genes Z4, Z5, Z6, Z10, and Z13 with a loss of imprinting, the expressed quantity of each of the imprinted genes Z4, Z5, Z6, Z10, and Z13 with a copy number variation, and the total expressed quantity of each of the imprinted genes Z4, Z5, Z6, Z10, and Z13 are graded into the following five grades:

Grade 0: applicable when any one or at least two of the following conditions are met: the expressed quantity of one of the imprinted genes Z4, Z5, Z6, Z10, and Z13 with a loss of imprinting is less than 10%, the expressed quantity of one of the imprinted genes Z4, Z5, Z6, Z10, and Z13 with a copy number variation is less than 1%, and the total expressed quantity of one of the imprinted genes Z4, Z5, Z6, Z10, and Z13 is less than 15%;

Grade I: applicable when any one or at least two of the following conditions are met: the expressed quantity of one of the imprinted genes Z4, Z5, Z6, Z10, and Z13 with a loss of imprinting is 10%-15%, the expressed quantity of one of the imprinted genes Z4, Z5, Z6, Z10, and Z13 with a copy number variation is 1%-2%, and the total expressed quantity of one of the imprinted genes Z4, Z5, Z6, Z10, and Z13 is 15%-20%;

Grade II: applicable when any one or at least two of the following conditions are met: the expressed quantity of one of the imprinted genes Z4, Z5, Z6, Z10, and Z13 with a loss of imprinting is 15%-20%, the expressed quantity of one of the imprinted genes Z4, Z5, Z6, Z10, and Z13 with a copy number variation is 2%-4%, and the total expressed quantity of one of the imprinted genes Z4, Z5, Z6, Z10, and Z13 is 20%-30%;

Grade III: applicable when any one or at least two of the following conditions are met: the expressed quantity of one of the imprinted genes Z4, Z5, Z6, Z10, and Z13 with a loss of imprinting is 20%-25%, the expressed quantity of one of the imprinted genes Z4, Z5, Z6, Z10, and Z13 with a copy number variation is 4%-6%, and the total expressed quantity of one of the imprinted genes Z4, Z5, Z6, Z10, and Z13 is 30%-40%; and

Grade IV: applicable when any one or at least two of the following conditions are met: the expressed quantity of one of the imprinted genes Z4, Z5, Z6, Z10, and Z13 with a loss of imprinting is greater than 25%, the expressed quantity of one of the imprinted genes Z4, Z5, Z6, Z10, and Z13 with a copy number variation is greater than 6%, and the total expressed quantity of one of the imprinted genes Z4, Z5, Z6, Z10, and Z13 is greater than 40%; and

the expressed quantity of the imprinted gene Z8 with a loss of imprinting, the expressed quantity of the imprinted gene Z8 with a copy number variation, and the total expressed quantity of the imprinted gene Z8 are graded into the following five grades:

Grade 0: applicable when any one or at least two of the following conditions are met: the expressed quantity of the imprinted gene Z8 with a loss of imprinting is less than 20%, the expressed quantity of the imprinted gene Z8 with a copy number variation is less than 2%, and the total expressed quantity of the imprinted gene Z8 is less than 15%;

Grade I: applicable when any one or at least two of the following conditions are met: the expressed quantity of the imprinted gene Z8 with a loss of imprinting is 20%-25%, the expressed quantity of the imprinted gene Z8 with a copy number variation is 2%-4%, and the total expressed quantity of the imprinted gene Z8 is 15%-20%;

Grade II: applicable when any one or at least two of the following conditions are met: the expressed quantity of the imprinted gene Z8 with a loss of imprinting is 25%-30%, the expressed quantity of the imprinted gene Z8 with a copy number variation is 4%-7%, and the total expressed quantity of the imprinted gene Z8 is 20%-30%;

Grade III: applicable when any one or at least two of the following conditions are met: the expressed quantity of the imprinted gene Z8 with a loss of imprinting is 30%-35%, the expressed quantity of the imprinted gene Z8 with a copy number variation is 7%-10%, and the total expressed quantity of the imprinted gene Z8 is 30%-40%; and

Grade IV: applicable when any one or at least two of the following conditions are met: the expressed quantity of the imprinted gene Z8 with a loss of imprinting is greater than 35%, the expressed quantity of the imprinted gene Z8 with a copy number variation is greater than 10%, and the total expressed quantity of the imprinted gene Z8 is greater than 40%.

9. A device for detecting a degree of benignity/malignancy of a lung tumor, wherein the device uses the model of any of claims 1-8 and comprises:

   (1) a sampling unit for obtaining a test sample;
   (2) a probe designing unit for designing a primer specific to the sequence of the imprinted gene, wherein the primer serves as a probe;
   (3) a detection unit for performing in-situ hybridization between the probe designed by the probe designing unit and the test sample; and
   (4) an analysis unit for analyzing expression of the imprinted gene through microscopic imaging;

   wherein the analysis unit calculates and grades, via the model of any of claims 1-8, the expressed quantity of the imprinted gene with a loss of imprinting and the expressed quantity of the imprinted gene with a copy number variation, and diagnoses the degree of benignity/malignancy of the lung tumor according to grading results of the expressed quantities of the imprinted gene with, respectively, a loss of imprinting and a copy number variation.

10. A method for detecting a degree of benignity/malignancy of a lung tumor, wherein the method uses the model of any of claims 1-8 or the device of claim 9 and comprises the steps of:

    (1) obtaining a test sample;
    (2) designing a primer specific to the sequence of the imprinted gene, wherein the primer serves as a probe;
    (3) performing in-situ hybridization between the probe designed in step (2) and the test sample; and
    (4) analyzing expression of the imprinted gene through microscopic imaging so as to diagnose the degree of benignity/malignancy of the lung tumor;

    wherein step (4) comprises calculating and grading, via the model of any of claims 1-8, the expressed quantity of the imprinted gene with a loss of imprinting, the expressed quantity of the imprinted gene with a copy number variation, and the total expressed quantity of the imprinted gene, and diagnosing the degree of benignity/malignancy of the lung tumor according to grading results of the expressed quantities of the imprinted gene with, respectively, a loss of imprinting and a copy number variation.

11. The method of claim 10, wherein the test sample in step (1) is tissue and/or cells obtained from a human.

12. The method of claim 10 or 11, wherein the test sample is any one, or a combination of at least two, of a paraffin section of a tissue, a lung needle biopsy sample, a biopsy sample obtained through bronchoscopy, a cell smear prepared from a bronchoalveolar lavage fluid, and a cell smear prepared from sputum.

13. The method of any of claims 10-12, wherein the in-situ hybridization uses an RNAscope in-situ hybridization method.

14. The method of any of claims 10-13, wherein the RNAscope in-situ hybridization method uses a single- or multiple-channel chromogenic reagent kit or a single- or multiple-channel fluorescent reagent kit, preferably a single-channel red/brown chromogenic reagent kit or a multiple-channel fluorescent reagent kit.

15. The method of any of claims 10-14, wherein the degree of benignity/malignancy of the lung tumor is classified as one of the following: benign tumor, potential lung cancer, early-stage lung cancer, intermediate-stage lung cancer, and advanced lung cancer.

**16.** The method of any of claims 10-15, wherein the degree of benignity/malignancy of the lung tumor is diagnosed as benign tumor when the expressed quantities of each of the imprinted genes Z1, Z3, Z4, Z5, Z6, Z8, Z10, Z11, Z13, and Z16 with, respectively, a loss of imprinting and a copy number variation are both graded as Grade 0; or when the expressed quantity of not more than one of the imprinted genes Z1, Z3, Z4, Z5, Z6, Z8, Z10, Z11, Z13, and Z16 with a loss of imprinting is graded as Grade I, and the expressed quantity of not more than one of the imprinted genes Z1, Z3, Z4, Z5, Z6, Z8, Z10, Z11, Z13, and Z16 with a copy number variation is graded as Grade I;

the degree of benignity/malignancy of the lung tumor is diagnosed as potential lung cancer when the expressed quantities of at least two of the imprinted genes Z1, Z3, Z4, Z5, Z6, Z8, Z10, Z11, Z13, and Z16 with a loss of imprinting are graded as Grade I; or when the expressed quantities of at least two of the imprinted genes Z1, Z3, Z4, Z5, Z6, Z8, Z10, Z11, Z13, and Z16 with a copy number variation are graded as Grade I; or when the expressed quantity of not more than one of the imprinted genes Z1, Z3, Z4, Z5, Z6, Z8, Z10, Z11, Z13, and Z16 with a loss of imprinting is graded as Grade II, and the expressed quantity of not more than one of the imprinted genes Z1, Z3, Z4, Z5, Z6, Z8, Z10, Z11, Z13, and Z16 with a copy number variation is graded as Grade II;

the degree of benignity/malignancy of the lung tumor is diagnosed as early-stage lung cancer when the expressed quantities of at least two of the imprinted genes Z1, Z3, Z4, Z5, Z6, Z8, Z10, Z11, Z13, and Z16 with a loss of imprinting are graded as Grade II; or when the expressed quantities of at least two of the imprinted genes Z1, Z3, Z4, Z5, Z6, Z8, Z10, Z11, Z13, and Z16 with a copy number variation are graded as Grade II; or when the expressed quantity of not more than one of the imprinted genes Z1, Z3, Z4, Z5, Z6, Z8, Z10, Z11, Z13, and Z16 with a loss of imprinting is graded as Grade III, and the expressed quantity of not more than one of the imprinted genes Z1, Z3, Z4, Z5, Z6, Z8, Z10, Z11, Z13, and Z16 with a copy number variation is graded as Grade III;

the degree of benignity/malignancy of the lung tumor is diagnosed as intermediate-stage lung cancer when the expressed quantities of at least two of the imprinted genes Z1, Z3, Z4, Z5, Z6, Z8, Z10, Z11, Z13, and Z16 with a loss of imprinting are graded as Grade III; or when the expressed quantities of at least two of the imprinted genes Z1, Z3, Z4, Z5, Z6, Z8, Z10, Z11, Z13, and Z16 with a copy number variation are graded as Grade III; or when the expressed quantity of not more than one of the imprinted genes Z1, Z3, Z4, Z5, Z6, Z8, Z10, Z11, Z13, and Z16 with a loss of imprinting is graded as Grade IV, and the expressed quantity of not more than one of the imprinted genes Z1, Z3, Z4, Z5, Z6, Z8, Z10, Z11, Z13, and Z16 with a copy number variation is graded as Grade IV; and

the degree of benignity/malignancy of the lung tumor is diagnosed as advanced-stage lung cancer when the expressed quantities of at least two of the imprinted genes Z1, Z3, Z4, Z5, Z6, Z8, Z10, Z11, Z13, and Z16 with a loss of imprinting are graded as Grade IV; or when the expressed quantities of at least two of the imprinted genes Z1, Z3, Z4, Z5, Z6, Z8, Z10, Z11, Z13, and Z16 with a copy number variation are graded as Grade IV.

**17.** A use of the model of any of claims 1-8 and/or the device of claim 9 in detecting a lung tumor.

**18.** A use of the model of any of claims 1-8 and/or the device of claim 9 in preparing or making a medicine or instrument for treating a lung tumor.

**19.** The use of claim 17 or 18, wherein a degree of benignity/malignancy of the lung tumor is classified as one of the following: benign tumor, potential lung cancer, early-stage lung cancer, intermediate-stage lung cancer, and advanced lung cancer.

**20.** The use of any of claims 17-19, wherein a degree of benignity/malignancy of the lung tumor is diagnosed as benign tumor when the expressed quantities of each of the imprinted genes Z1, Z3, Z4, Z5, Z6, Z8, Z10, Z11, Z13, and Z16 with, respectively, a loss of imprinting and a copy number variation are both graded as Grade 0; or when the expressed quantity of not more than one of the imprinted genes Z1, Z3, Z4, Z5, Z6, Z8, Z10, Z11, Z13, and Z16 with a loss of imprinting is graded as Grade I, and the expressed quantity of not more than one of the imprinted genes Z1, Z3, Z4, Z5, Z6, Z8, Z10, Z11, Z13, and Z16 with a copy number variation is graded as Grade I;

the degree of benignity/malignancy of the lung tumor is diagnosed as potential lung cancer when the expressed quantities of at least two of the imprinted genes Z1, Z3, Z4, Z5, Z6, Z8, Z10, Z11, Z13, and Z16 with a loss of imprinting are graded as Grade I; or when the expressed quantities of at least two of the imprinted genes Z1, Z3, Z4, Z5, Z6, Z8, Z10, Z11, Z13, and Z16 with a copy number variation are graded as Grade I; or when the expressed quantity of not more than one of the imprinted genes Z1, Z3, Z4, Z5, Z6, Z8, Z10, Z11, Z13, and Z16 with a loss of imprinting is graded as Grade II, and the expressed quantity of not more than one of the imprinted genes Z1, Z3, Z4, Z5, Z6, Z8, Z10, Z11, Z13, and Z16 with a copy number variation is graded as Grade II;

the degree of benignity/malignancy of the lung tumor is diagnosed as early-stage lung cancer when the expressed quantities of at least two of the imprinted genes Z1, Z3, Z4, Z5, Z6, Z8, Z10, Z11, Z13, and Z16 with a loss of imprinting are graded as Grade II; or when the expressed quantities of at least two of the imprinted genes Z1, Z3, Z4, Z5, Z6, Z8, Z10, Z11, Z13, and Z16 with a copy number variation are graded as Grade II; or when the expressed quantity of not more than one of the imprinted genes Z1, Z3, Z4, Z5, Z6, Z8, Z10, Z11, Z13, and Z16 with a loss of imprinting is graded as Grade III, and the expressed quantity of not more than one of the imprinted genes Z1, Z3, Z4, Z5, Z6, Z8, Z10, Z11, Z13, and Z16 with a copy number variation is graded as Grade III;

the degree of benignity/malignancy of the lung tumor is diagnosed as intermediate-stage lung cancer when the expressed quantities of at least two of the imprinted genes Z1, Z3, Z4, Z5, Z6, Z8, Z10, Z11, Z13, and Z16 with a loss of imprinting are graded as Grade III; or when the expressed quantities of at least two of the imprinted genes Z1, Z3, Z4, Z5, Z6, Z8, Z10, Z11, Z13, and Z16 with a copy number variation are graded as Grade III; or when the expressed quantity of not more than one of the imprinted genes Z1, Z3, Z4, Z5, Z6, Z8, Z10, Z11, Z13, and Z16 with a loss of imprinting is graded as Grade IV, and the expressed quantity of not more than one of the imprinted genes Z1, Z3, Z4, Z5, Z6, Z8, Z10, Z11, Z13, and Z16 with a copy number variation is graded as Grade IV; and

the degree of benignity/malignancy of the lung tumor is diagnosed as advanced lung cancer when the expressed quantities of at least two of the imprinted genes Z1, Z3, Z4, Z5, Z6, Z8, Z10, Z11, Z13, and Z16 with a loss of imprinting are graded as Grade IV; or when the expressed quantities of at least two of the imprinted genes Z1, Z3, Z4, Z5, Z6, Z8, Z10, Z11, Z13, and Z16 with a copy number variation are graded as Grade IV.

FIG. 1

Grade 0

FIG. 2(a)

Grade I

FIG. 2(b)

Grade II

FIG. 2(c)

Grade III

FIG. 2(d)

Grade IV

FIG. 2(e)

FIG. 3(a)

FIG. 3(b)

FIG. 3(c)

FIG. 3(d)

FIG. 3(e)

FIG. 3(f)

FIG. 4(a)

FIG. 4(b)

FIG. 4(c)

FIG. 4(d)

FIG. 4(e)

FIG. 4(f)

FIG. 4(g)

FIG. 4(h)

FIG. 4(i)

FIG. 4(j)

FIG. 5(a)

FIG. 5(b)

FIG. 5(c)

FIG. 5(d)

FIG. 5(e)

FIG. 5(f)

FIG. 5(g)

FIG. 5(h)

FIG. 5(i)

FIG. 5(j)

## INTERNATIONAL SEARCH REPORT

International application No.

**PCT/CN2019/095415**

### A. CLASSIFICATION OF SUBJECT MATTER

C12Q 1/6886(2018.01)i

According to International Patent Classification (IPC) or to both national classification and IPC

### B. FIELDS SEARCHED

Minimum documentation searched (classification system followed by classification symbols)

C12Q

Documentation searched other than minimum documentation to the extent that such documents are included in the fields searched

Electronic data base consulted during the international search (name of data base and, where practicable, search terms used)

DWPI, SIPOABS, CPRSABS, CPEA, TWABS, CNABS, JPABS, CNTXT, USTXT, WOTXT, EPTXT, CNKI, 万方, WANFANG, ISI Web of Science, Elsevier Science: gnas, grb10, snrpn, snurf, peg10, igf2r, mest, plagl1, dcn, gatm, sgce, 印记基因, 分级, 等级, 肺癌, 肺, 肿瘤, 模型, 杂交, 探针, 拷贝数, 表达量, imprinting, imprinted, grade, grading, classify, lung, cancer, tumor, carcinoma, hybridization, model, copy number, expression

### C. DOCUMENTS CONSIDERED TO BE RELEVANT

| Category* | Citation of document, with indication, where appropriate, of the relevant passages | Relevant to claim No. |
|---|---|---|
| Y | WO 9524503 A1 (RAPAPORT ERICH ET AL.) 14 September 1995 (1995-09-14) see description, page 5, line 1 to page 7, line 24, and page 9, line 6 to page 10, line 9 | 1-20 |
| Y | WO 2016154330 A1 (WHITEHEAD INSTITUTE FOR BIOMEDICAL RESEARCH) 29 September 2016 (2016-09-29) see description, paragraphs [0005]-[0011] | 1-20 |
| Y | WO 2007097741 A1 (THE TRUSTEES OF COLUMBIA UNIVERSITY IN THE CITY OF NEW YORK ET AL.) 30 August 2007 (2007-08-30) see description, page 7, lines 1-5, and page 2, lines 4-10 | 1-20 |

☐ Further documents are listed in the continuation of Box C.　　☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **10 October 2019** | **16 October 2019** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088 China** | |
| Facsimile No. **(86-10)62019451** | Telephone No. |

Form PCT/ISA/210 (second sheet) (January 2015)

## INTERNATIONAL SEARCH REPORT
### Information on patent family members

International application No.

**PCT/CN2019/095415**

| Patent document cited in search report | | | Publication date (day/month/year) | Patent family member(s) | | | Publication date (day/month/year) |
|---|---|---|---|---|---|---|---|
| WO | 9524503 | A1 | 14 September 1995 | EP | 0759092 | A1 | 26 February 1997 |
| | | | | DE | 69526855 | T2 | 28 November 2002 |
| | | | | IL | 108879 | D0 | 24 June 1994 |
| | | | | EP | 0759092 | B1 | 29 May 2002 |
| | | | | US | 5955273 | A | 21 September 1999 |
| | | | | DE | 69526855 | D1 | 04 July 2002 |
| | | | | AU | 1893095 | A | 25 September 1995 |
| | | | | ES | 2177653 | T3 | 16 December 2002 |
| | | | | IL | 108879 | A | 31 August 2000 |
| WO | 2016154330 | A1 | 29 September 2016 | US | 2016340749 | A1 | 24 November 2016 |
| | | | | US | 2019194764 | A1 | 27 June 2019 |
| | | | | US | 10023922 | B2 | 17 July 2018 |
| WO | 2007097741 | A1 | 30 August 2007 | | None | | |

Form PCT/ISA/210 (patent family annex) (January 2015)